# EUROPEAN PATENT SPECIFICATION

(11) **EP 2 865 753 B1**
(45) Date of publication and mention of the grant of the patent: **29.03.2017**
(21) Application number: 14190020.9
(22) Date of filing: 23.10.2014
(51) Int. Cl.: C12N 15/10, C12Q 1/68, C12N 15/115

(54) **Magnetic-assisted rapid aptamer selection method for generating high affinity DNA aptamer**
Magnetisch unterstütztes, schnelles Aptamerauswahlverfahren zur Erzeugung eines hochaffinen DNA-Aptamers
Procédé de sélection rapide d'aptamères à assistance magnétique pour générer un aptamère d'ADN à haute affinité

(30) Priority: 28.10.2013 US 201314065382
(43) Date of publication of application: 29.04.2015
(73) Proprietor: Hong, Chin-Yih, Changhua 51065 (TW)
(72) Inventor: Hong, Chin-Yih, Changhua 51065 (TW); Lai, Chi-Ching, Taichung City 414 (TW)
(74) Representative: Becker Kurig Straus

(56) References cited:
- WO-A1-99/54506
- YANG S Y ET AL: "Magnetically enhanced high-specificity virus detection using bio-activated magnetic nanoparticles with antibodies as labeling markers", JOURNAL OF VIROLOGICAL METHODS, ELSEVIER BV, NL, vol. 164, no. 1-2, 1 March 2010 (2010-03-01), pages 14-18, XP026925454, ISSN: 0166-0934, DOI: 10.1016/J.JVIROMET.2009.11.016 [retrieved on 2009-11-26]
- LAI JI-CHING ET AL: "Magnetic-Assisted Rapid Aptamer Selection (MARAS) for Generating High-Affinity DNA Aptamer Using Rotating Magnetic Fields", July 2014 (2014-07), ACS COMBINATORIAL SCIENCE, VOL. 16, NR. 7, PAGE(S) 321-327, XP002734453, ISSN: 2156-8952(print) * the whole document *

## Description

### BACKGROUND

### Technical Field

The present invention relates to a selection method. Particularly, the present invention relates to a magnetic-assisted rapid aptamer selection (MARAS) method.

### Related Art

Systematic evolution of ligands by exponential enrichment (SELEX) is an in vitro selection method for an isolation of DNA/RNA sequences from large combinatorial libraries of random sequences (naive libraries) and the isolated sequences bind to molecules, based on the ability of DNA/RNA to fold into three dimensional shapes which rivals those of molecules. A successful SELEX product is called 'aptamer' whose structures consist of binding pockets that could bind with high specificity and affinity to a variety of molecular targets, diversified from micro-molecules (such as organic molecules, ions, peptides, proteins, nucleic acids), macro-molecules to even whole cells, viruses, parasites or tissues. Once the sequence of aptamer is identified for molecular targets, the entire aptamer can be produced by chemical synthesis. Furthermore, aptamers modified with functional groups can increase their stability in various biological applications, but being harmful for nucleic acids. Compared with traditional antibody products, one advantage of aptamers lies in that hybridoma and animal sacrifice are not required. Aptamers not only have the potential to be an excellent tool to target pathogenic and malignant cells or tissues and substitute antibodies but also can be applied on purification, diagnostics, biosensors and anti-infectious agents. However, unlike commercially available antibodies, it is difficult to obtain a suitable aptamer for further applications.

SELEX involves multiple rounds of alternating incubation, separation, elution, amplification and purification steps, and these steps may be summarized as: (1) a naive DNA library is mixed with a target molecule; (2) DNA bound to the target molecule is separated from unbound DNA; (3) the bound DNA is eluted from the target molecule; (4) the eluted DNA is amplified by PCR to produce an aptamer-enriched DNA library; and (5) the single strand oligonucleotides are purified from the PCR product. This procedure is repeated several rounds starting with the enriched library obtained in the previous round. Typically, at least five to fifteen SELEX selection rounds are performed until no further enrichment of the functional nucleic acid species is detectable. Multiple rounds of selection to isolate aptamers with sufficient specificity and binding affinity (e.g., nanomolar dissociation constant, Kd) are needed. In general, SELEX is a time-consuming and lengthy protocol. Even for automated SELEX procedures where the selection of aptamers may be completed in several days, it still requires large amounts of preparations and high operation costs. In addition, several functional strategies were proposed to simplify this lengthy procedure, including capillary electrophoresis (CE-SELEX) and microfluidics SELEX (M-SELEX). However, CE-SELEX is less effective to screen aptamers which bind with small molecules, while M-SELEX requires expensive microfludic instruments.

As the aptamers are potential in many aspects, it is desirable to develop an efficient screen protocol simple enough to rapidly screen suitable aptamers with high affinity and specificity for their applications.

### SUMMARY

The present disclosure provides an aptamer selection protocol utilizing biofunctionalized magnetic particles to screen the target-bound oligonucleotides from the DNA library. Such protocol, magnetic-assisted rapid aptamer selection (MARAS), is an efficient protocol to obtain high affinity aptamers. Furthermore, such aptamer selection protocol may be completed within an hour, excluding the material preparation and the post analysis which are also needed for other aptamer selection protocol, and may be automated with high throughput.

The present disclosure provides an aptamer selection protocol. At first, a sample comprising at least a random sequence library having a plurality of oligonucleotide sequences and a plurality of magnetic nanoparticles or micro-particles having target molecules joined thereon is provided. A portion of the plurality of oligonucleotide sequences is bound to the plurality of magnetic nanoparticles or micro-particles having the target molecules joined thereon. A magnetic-assisted screening is performed by applying an oscillation magnetic field to the sample to isolate the portion of the plurality of oligonucleotide sequences bound to the plurality of magnetic nanoparticles or micro-particles having the target molecules joined thereon. Then, a negative selection is performed to the isolated portion of the plurality of oligonucleotide sequences bound to the plurality of magnetic nanoparticles or micro-particles without having the target molecules joined thereon. Then a magnetic separation is performed by using a magnetic stand to isolate aptamers specific to the target molecules.

The present disclosure also provides an aptamer selection protocol. At first, a sample comprising at least a random sequence library having a plurality of oligonucleotide sequences and a plurality of magnetic nanoparticles or micro-particles having target molecules joined thereon is provided. The target molecules are joined to the plurality of magnetic nanoparticles or micro-particles through first components attached to the plurality of magnetic nanoparticles or micro-particles and second components attached to the target molecules. A magnetic-assisted screening is performed to provide a competitive mechanism by applying an oscillation magnetic field. A negative selection is performed to the sample using a plurality of magnetic nanoparticles or micro-particles having the first components attached thereon to isolate a portion of the plurality of oligonucleotide sequences not bound to the plurality of magnetic nanoparticles or micro-particles having the first components attached thereon. Then a magnetic separation is performed by using a magnetic stand to isolate the portion of the plurality of oligonucleotide sequences not bound to the plurality of magnetic nanoparticles or micro-particles having the first components attached thereon to select aptamers specific to the target molecules.

According to embodiments of the present disclosure the oscillation magnetic field is a rotating magnetic field.

According to embodiments of the present disclosure the process of performing the magnetic-assisted screening further comprises performing a plurality of rounds of the magnetic-assisted screening by applying a rotating magnetic field of a fixed magnetic field strength and at a variety of magnetic field frequencies, wherein the magnetic field frequency used in each round of the magnetic-assisted screening is different from that of the other round of the magnetic-assisted screening.

According to embodiments of the present invention, the rotating magnetic field is applied at 14 gauss with the magnetic field frequencies varying from 2 Hz to 27 KHz.

According to embodiments of the present invention, the oscillation magnetic field is an alternating magnetic field.

According to embodiments of the present disclosure the process of performing a magnetic-assisted screening further comprises performing a plurality of rounds of the magnetic-assisted screening by applying an alternating magnetic field of a fixed magnetic field strength and at a variety of magnetic field frequencies, wherein the magnetic field frequency used in each round of the magnetic-assisted screening is different from that of the other round of the magnetic-assisted screening.

According to embodiments of the present invention, the alternating magnetic field is applied at 25 gauss with the magnetic field frequencies varying from 2 Hz to 200 KHz.

According to embodiments of the present disclosure the process of performing a magnetic-assisted screening further comprises performing a plurality of rounds of the magnetic-assisted screening by applying an alternating magnetic field of a fixed magnetic field frequency and at a variety of magnetic field strengths, wherein the magnetic field strength used in each round of the magnetic-assisted screening is different from that of the other round of the magnetic-assisted screening.

According to embodiments of the present invention, the alternating magnetic field is applied of 100 KHz with the magnetic field strengths varying from 12.5 gauss to 400 gauss.

According to embodiments of the present disclosure the target is C-reactive protein, the first component is streptavidin and the second component is biotin.

In order to make the aforementioned and other features and advantages of the disclosure comprehensible, several exemplary embodiments accompanied with figures are described in detail below.

### BRIEF DESCRIPTION OF THE DRAWINGS

The accompanying drawings are included to provide a further understanding of the disclosure, and are incorporated in and constitute a part of this specification. The drawings illustrate embodiments of the disclosure and, together with the description, serve to explain the principles of the disclosure.
Figure 1A is a schematic diagram of the experimental setting for the rotating magnetic field magnetic-assisted rapid aptamer selection (RO-MARAS) protocol according to one embodiment of the present invention.
Figure 1B is a schematic diagram of the experimental setting for the alternating magnetic field magnetic-assisted rapid aptamer selection (AC-MARAS) protocol according to one embodiment of the present invention.
Figure 2 schematically illustrates the process flow for the frequency dependent analysis of RO-MARAS according to one embodiment of the present invention.
Figure 3 shows the relationship of the magnetic field frequency applied in RO-MARAS and dissociation constants (Kd) of the selected 20N aptamers screened by RO-MARAS according to one embodiment of the present invention.
Figure 4 shows the relationship of the magnetic field frequency applied in AC-MARAS and dissociation constants (Kd) of the selected 20N aptamers screened by AC-MARAS according to one embodiment of the present invention.
Figure 5 shows the relationship of the magnetic field strength used in AC-MARAS and dissociation constants (Kd) of the selected 20N aptamers screened by AC-MARAS according to one embodiment of the present invention.

### DETAILED DESCRIPTION OF DISCLOSED EMBODIMENTS

The present invention relates to an aptamer selection protocol using biofunctionalized magnetic particles to screen the target-bound oligonucleotides from the DNA library. As C-reactive protein (CRP) is a common indicator of inflammation, heart attack, stroke and cardiovascular diseases, CRP is used as the target in the following experiments and the bio-functionalized magnetic particles are coated with CRP thereon for experimental purposes. Herein, the CRP coated biofunctionalized magnetic nanoparticles (CRP-MNP) and CRP coated biofunctionalized magnetic microparticles (CRP-MMP) were driven by a magnetic torque which is generated by an externally applied oscillation magnetic field. The aptamer selection protocol of the present invention is a magnetic-assisted rapid aptamer selection (MARAS) protocol.

Before performing the MARAS protocols, it is required to prepare the materials. The material preparation includes preparing the random sequence library, preparing the target and incubating the target with the random sequence library. These preparation steps will be summarized in the following sections.

### Oligonucleotide Library and Primers

Three types of randomized oligonucleotide sequences 60N, 40N and 20N were used as the random sequence libraries. The randomized oligonucleotide sequences are written as: 5'-AGCAGCACAGAGGTCAGATG-**Xn**-CCTATGCGTGCTACCGTGAA-3', with the randomized (N)-mer midsection **Xn**, while **n** represents any integer ranging from 10 to 100 and **Xn** means the nucleotide sequence of **n**-mer nucleotides. For example, randomized oligonucleotide sequences 60N, 40N or 20N may be the nucleic acid sequences having the randomized X₆₀, X₄₀ or X₂₀ midsection, and the randomized X₆₀, X₄₀ or X₂₀ midsection respectively represent randomized 60-mer sequences (sequences of 60 nucleotides), randomized 40-mer sequences or randomized 20-mer sequences. The randomized oligonucleotide sequences are synthesized at 1 mM scale and PAGE purified (purchased from Invitrogen Life Technologies, Grand Island, NY, USA). One set of primers, including Lab-forward 5'-AGCAGCACAGAGGTCAGATG-3' (SEQ ID NO.1) and Lab-reverse 5'-TTCACGGTAGCACGCATAGG-3' (SEQ ID NO. 2), was used to anneal the 5' and 3' degenerating region of the library during the PCR amplification. 5'-biotin labeled primer: Lab-biotin-R, with the same sequence described above was used to isolate forward single strand from the double strand PCR product. It is noted that if the reverse single strand aptamer is preferred, then Lab-biotin-F primer can be used to substitute the Lab-biotin-R for the single strand purification. The universal T7 primer (T7: 5'-TAATACGACTCACTATAGGG-3' (SEQ ID NO. 3)) was used to sequence the nucleotide of the selected aptamer.

### CRP Coated Biofunctionalized Magnetic Particles

The reagent containing streptavidin coated magnetic nanoparticle (SA-MNPs) used in this study was purchased from Magqu (Magqu, Taipei, Taiwan). The average hydrodynamic diameter of the SA-MNPs in the reagent was 50 nm with a concentration of 0.3 emu/g. The magnetic nanoparticles were bio-functionalized by coating streptavidin on the outmost surface of nanoparticles and were dispersed in PBS (pH=7.4). The reagent containing streptavidin coated magnetic micro-particle (SA-MMPs) was also purchased from Magqu and the average hydrodynamic diameter of the SA-MMPs was around 3 µm with a concentration of 8 mg/ml. Other alternatives, such as dextran, mono or poly-antibodies, or amino groups, may be used to bio-functionalize magnetic particles. The purity of the human CRP used in this study was greater than 99% (purchased from MYBIOSOURCE, San Diego USA). The biotinylation kit (Biotin Labeling Kit-NH₂) was purchased from Abnova (Abnova, Taipei, Taiwan). In this study, 600 µg of pure CRP protein was used and the biotinylated CRP protein was prepared according to the manufacturer's instructions. Afterward, a total of 250 µg biotinylated CRP protein was mixed with 50 µl of SA-MNP reagent or SA-MMP reagent, and incubated overnight at 4°C. The high-affinity binding between streptavidin and biotin ensured the conjugation between the magnetic particles and biotinylated CRP protein. Herein, the target CRP is attached to the magnetic particles (MNPs or MMPs) through the binding of streptavidin-biotin, and streptavidin-biotin may be considered as a conjugation pair. Then the incubated solution was subjected to magnetic separation to remove the unbound biotinylated CRP by using a magnetic stand (Magqu). The CRP-MNPs and CRP-MMPs were re-suspended in 50 µl PBS-T (50 mM K₂HPO₄, pH 7.5, 150 mM NaCl, 0.05% Tween-20) to respectively form CRP-labeled magnetic nanoparticle reagent (CRP-MNP reagent) and CRP-labeled magnetic micro-particle reagent (CRP-MMP reagent), and stored at 4°C. The final concentration of biotinylated CRP in CRP-MNP reagent is 188.47±1.30 ng/µl and in CRP-MMP reagent is 143.95±4.29 ng/µl, determined by Bradford assay (Bio-Rad, Hercules, CA. USA). Before use, both CRP-MNP reagent and CRP-MMP reagent were washed three times with the binding buffer (BD buffer: 50 mM NaH₂PO₄, pH 8.0, 150 mM NaCl, 5 mM KCl, 2 mM MgCl₂, 0.005% (v/v) Tween-20). During each washing step, the magnetic stand was used to collect the CRP-MNPs and CRP-MMPs. Similarly, if the SA-MNPs and SA-MMPs were needed, then streptavidin coated magnetic nanoparticle reagent (SA-MNP reagent) and streptavidin coated magnetic micro-particle reagent (SA-MMP reagent) were washed three times with binding buffer and the SA-MNPs and SA-MMPs were collected using the magnetic stand.

### Experimental Setting of MARAS

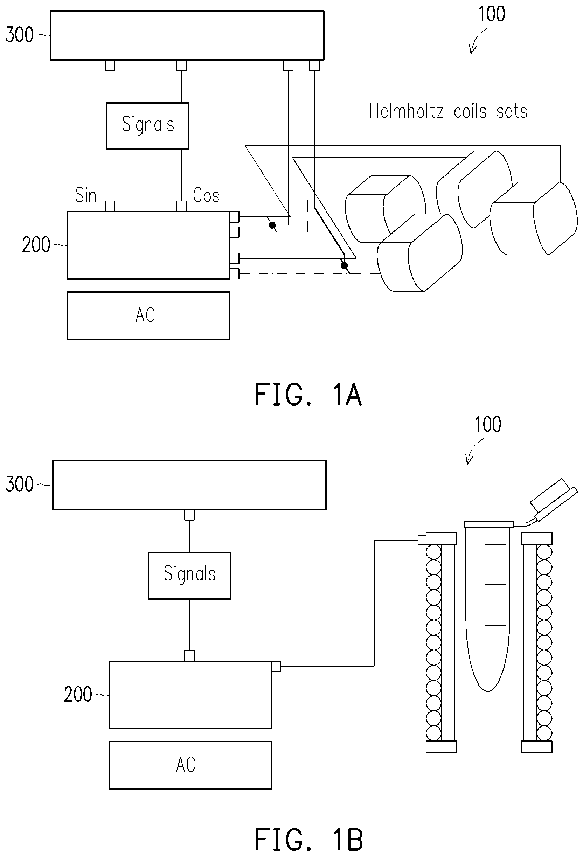

The experimental setting for performing MARAS protocol was described as below. The experimental setting includes at least two sets of coils 100 for generating an oscillation magnetic field, a power amplifier 200, a signal generator 300 and is operated with the LABVIEW computer program. The oscillation magnetic fields used in MARAS may be either a rotating magnetic field as in the case of rotating magnetic field-MARAS (RO-MARAS) or an alternating magnetic field as in the case of alternating magnetic field-MARAS (AC-MARAS). For RO-MARAS, the rotating magnetic field was generated by two sets of Helmholtz coils placed orthogonally. The LABVIEW program, via a NI BNC-2110 capture box, was used to send two signals, cos(ωt) and sin(ωt), into a two-channel power amplifier (HCA3030D). These two signals were then amplified equally, which drove two sets of coils simultaneously to produce a rotating magnetic field. The experimental setting is schematically shown in Fig 1A. In the figures, the sample was placed at the intersection of the central lines of two sets of Helmholtz coils. However, other alternative setting may also be used to generate the rotating magnetic field. For AC-MARAS, the magnetic field was generated by a single excitation solenoid driven by a current generator unit, as schematically shown in Fig. 1B, in which the sample was placed inside the solenoid. It is noted that the setting of Fig. 1A can also be used for AC-MARAS if the computer program send only one signal to one set of Helmholtz coil to generate the AC magnetic field. Furthermore, other alternative setting may also be used to generate the alternating magnetic field. Moreover, other types of oscillation magnetic field may be also applicable, such as an elliptical magnetic field which can be generated by using different amplification factors of the power amplifier (200) for the sine and cosine signal from the signal generator (300).

DNA aptamers are oligonucleic acid sequences that bind to a specific target molecule, and the aptamers are usually obtained by selecting them from a large random sequence pool. Following the MARAS protocols, the selected aptamers screened by the MARAS protocols may be subjected to the post analyses, such as cloning, PCR amplification and binding affinity calculation (in terms of dissociation constants).

### MARAS protocols

The MARAS protocols may be summarized as the following processes: providing a sample comprising at least a random sequence library having a plurality of oligonucleotide sequences and a plurality of magnetic nanoparticles or micro-particles having target molecules joined thereon; performing a magnetic-assisted screening by applying an oscillation magnetic field to the sample; performing a magnetic separation to collect the bound mixture consisting of the oligonucleotide sequences bound to the plurality of magnetic nanoparticles or micro-particles having target molecules joined thereon; dispersing the bound mixture in binding buffer; heating the solution to elute the plurality of oligonucleotide sequences from the plurality of magnetic nanoparticles or micro-particles having the target molecules joined thereon; performing a magnetic separation by using a magnetic stand to collect the supernatant and to remove the plurality of magnetic nanoparticles or micro-particles having the target molecules joined thereon; heating the collected supernatant to 95°C for 5 minutes and snap cooled at 4°C; performing a negative selection to remove aptamers which bound to the plurality of magnetic nanoparticles or micro-particles without having target molecules joined thereon and to select aptamers specific to the target molecules; and performing a magnetic separation to collect the supernatant having the plurality of oligonucleotide sequences specifically to the target molecules.

### Cloning and Sequencing Analysis of Selected Aptamers

The supernatants which were collected from each MARAS experimental step were precipitated with 1 ml of 100% cold alcohol and diluted by 100 µl ddH₂O for a following PCR amplification. The collected supernatants were subsequently amplified by PCR with Lab-F and Lab-R primers. The PCR reaction which contained 1.25 U of DNA polymerase (Invitrogen), 0.1 mM dNTPs, 0.5 mM MgSO₄, 0.5 nM primers, was performed under the following conditions: 5 minutes at 95°C; 35 cycles of 40 seconds at 95°C, 40 seconds at 60°C, 40 seconds at 72°C; and 10 minutes at 72°C. The PCR product was purified by using a DNA Extraction Miniprep System (Viogene, Taipei, Taiwan). The purified product was sub-cloned into a pGEM-T Easy vector (Promega, Madison, WI, USA). The cloning procedure was performed according to the manufacturer's instruction. The plasmids of picked up colonies were purified by using a High-Speed Plasmid Mini Kit (Geneaid, Taipei, Taiwan). The plasmids were sequenced by using an Applied Biosystems PRISM 3730 DNA automatic sequencer and a Big Dye terminator cycle sequencing kit (Foster City, CA, USA).

### Estimation of Aptamer Binding Affinity by Real-Time Quantitative PCR

The affinity of the aptamers to the target CRP was described by the equilibrium dissociation constant (Kd), which was measured by real-time PCR. For each plasmid picked from the cloning experiments, 10 ng aptamer clone plasmid was used as PCR template to generate double strand DNA (dsDNA) with Lab-biotin-R and Lab-F primers. The PCR condition and procedure were as described above. After the completion of PCR amplification, the PCR product was mixed with SA-MNPs, obtained from washing 5 µl of SA-MNP reagent. Forward single strand aptamers (non-biotinylated strand) were separated from the immobilized complementary strand by being incubated with 0.15 N of fresh NaOH for five minutes. The bound SA-MNPs were removed by the magnetic stand. An equal amount of 0.15 N of HCl was added to the collected supernatant to adjust the final pH to 7.0, after which the forward ssDNA was precipitated with 1 ml of 100% ice-cold alcohol. It must be mentioned that reverse single strand aptamers can also be generated by eluting the bound mixture at 94°C then performing a magnetic separation. Moreover, the forward and reverse single strand aptamers can also be isolated by using Lab-biotin-F and Lab-R primers during PCR amplification. The concentration of the single strand aptamers was determined with a NanoDrop 2000c spectrophotometer (Thermo Fisher Scientific, Wilmington, DE, USA). A series of progressively diluted aptamers (200 nM to 1.5625 nM) in 20 µl of BD buffer were heated to 95°C for 5 minutes and cooled at 4°C for the formation of secondary structures. Partial diluted aptamers was retained as an input control (input). CRP-MNPs, obtained from washing 5 µl of CRP-MNP reagent, were added into each micro-tube containing diluted aptamers and incubated for 30 minutes at room temperature. The bound CRP-MNPs were washed twice with 100 µl of BD buffer. The bound aptamers were eluted from the CRP-MNPs by heating at 94°C for 10 minutes in final volume 20 µl of ddH₂O. The CRP-MNPs in the solution were removed with a magnetic stand, and the supernatants were collected. Both the input controls and eluted aptamers were precipitated with 1 ml of 100% ice-cold alcohol. The input control and eluted aptamers were dissolved in test tubes filled with 100 µl ddH₂O, separately. The quantities of the aptamers in each test tube, including input control tube and eluted aptamer tubes were calculated by real-time quantitative PCR (q-PCR). q-PCR was performed with MicroAmp optical 96-well reaction plates and the threshold cycle (ct) value was calculated automatically using the maximum correlation coefficient approach with StepOnePlus Real-Time PCR Systems software, version 2.0 (Applied Biosystems). The mixture for each q-PCR run was 10 µl containing 2.5 µl of SYBR Green PCR master mix (Applied Biosystems) and 0.5 nM of primer Lab forward and Lab reverse. The reaction condition was as follows: 95°C for 3 minutes; 40 cycles at 94°C for 30 seconds, 60°C for 30 seconds, and 72°C for 30 seconds. The concentrations of the aptamers in the input control and eluted aptamers were calculated, using 200 nM aptamers as indicative of maximum binding. The Kd value of the selected aptamer was then determined by fitting a saturation binding curve based on the experimental data via a curve fitting program, CurveExpert1.3 (from curveexpert.webhop.net).

### Parameter-Dependence of MARAS Procedure

One nanomole of the 20N randomized oligonucleotide library was diluted to 10 µl with BD buffer in a micro-tube, heated to 95°C for 5 minutes and snap cooled at 4°C. The CRP-MNPs, obtained from washing 5 µl of CRP-MNP reagent, were added into the micro-tube and was incubated for 30 minutes at room temperature. The unbound oligonucleotide sequences were removed as the oligonucleotide sequences bound to the CRP-MNPs are attracted to a magnetic stand, and the bound mixture was washed three times with 1 ml of BD buffer. 100 µl BD buffer was added to re-suspend the bound mixtures in the micro-tube as the sample. The applied magnetic field was either a rotating (RO-MARAS) or an alternating (AC-MARAS) magnetic field. The strength of the magnetic field was calibrated by a gauss meter at the sample location.

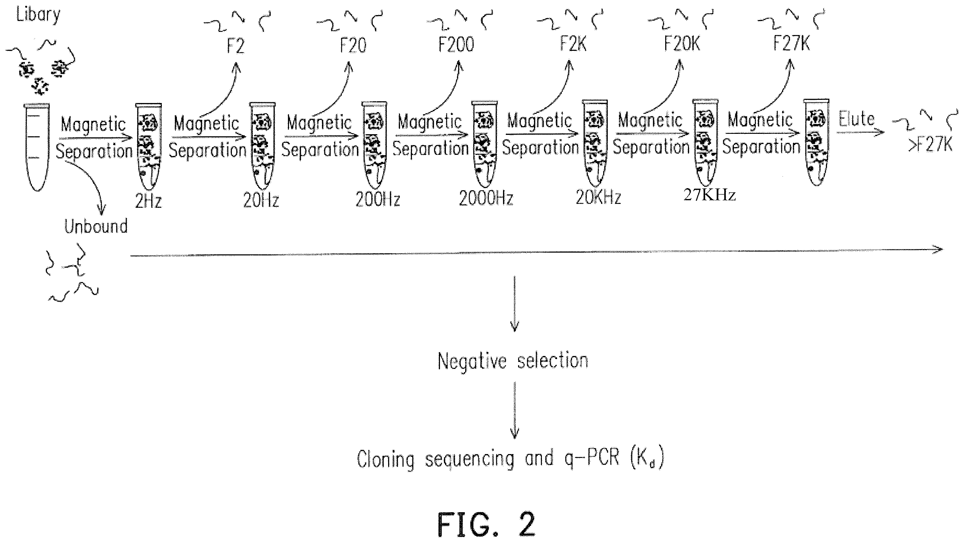

For a frequency dependent study of RO-MARAS, the bound mixtures (sample) were subjected to a rotating magnetic field with a constant magnetic field strength (14 gauss) and varying in the magnetic field frequency (e.g.: 2 Hz, 20 Hz, 200 Hz, 2000 Hz, 20 KHz and 27 KHz) for magnetic-assisted screening. Multiple rounds of magnetic-assisted screening are performed to isolate (select) aptamers with different binding affinity to the target molecules. Figure 2 schematically illustrates the process flow for the frequency dependent analysis of RO-MARAS according to one embodiment of the present invention. In this example, due to the power amplifier used, the highest frequency that can be reached is 27 KHz with field strength of 14 gauss for RO-MARAS. Initially, the sample was subjected to a magnetic field of 2 Hz for 10 minutes and was stirred every 2.5 minutes by pipetting in order to avoid agglomeration due to the action of magnetic field on magnetic nanoparticles. The purpose of applying an oscillation magnetic field to the sample is to generate a competitive mechanism on the binding among the oligonucleotide sequences and the CRP-MNPs. As the sample is subjected to an oscillation magnetic field, the bound mixtures of the sample are subjected to a driving torque produced by the interaction of the magnetic dipole of magnetic particle of the sample and the externally applied magnetic field, which induces rotational or oscillational motions of magnetic particle in the solution, and a dissipative torque due to the motion of magnetic particles in an aqueous solution. These two counter torques generate a stretch force on the binding among the bound oligonucleotide sequences and CRP-MNPs. The sequences bound to the CRP-MNPs will not dissociate until the stretch force counteracts the binding strength of the sequences toward the CRP-MNPs. The enhancement of the competitive mechanism can be achieved by increasing these two torques, by means of increasing the frequency and the strength of the oscillation magnetic field and the size of the magnetic particle. In this example, as the frequency of the oscillation magnetic field increases, the phase lag between the dipole moment of magnetic particle and the field direction increases, resulting in the increase of the driving torque which is a vector produce of the dipole moment of the magnetic particle and the applied magnetic field. Meanwhile, the dissipative torque also increases due to the higher angular velocity of magnetic particle which increases as the frequency of the driving magnetic field increases. Thus the stretch force can be enhanced by increasing the frequency of the externally applied oscillation magnetic field. The first round of the magnetic screening was performed at the field frequency of 2 Hz and the dissociated (unbound) nucleotide sequences in the supernatant was collected by using the magnetic stand. Based on the 20N randomized nucleotide sequence library, under the rotating magnetic field (RO) with field strength of 14 gauss and at the field frequency of 2 Hz, 20 Hz, 200 Hz, 2000 Hz, 20 KHz and 27 KHz, these collected unbound nucleotide sequences were named as 20N RO F2, F20, F200, F2K, F20K, F27K sequences, respectively, with individual reference numbers (as listed in Table 1). Consequently, 100 µl BD buffer was added to re-suspend the retained bound mixture and the mixture was subsequently subjected to a magnetic field with the next frequency 20 Hz as shown in Fig. 2. The aforementioned process was repeated until a magnetic field with final frequency (27 KHz) was applied. After the application of the magnetic field of the final frequency, the last round of magnetic separation was performed to separate supernatant and the bound mixture was retained. Finally, 100 µl BD buffer was added to the retained bound mixture and heated to 95°C for 5 minutes to elute the aptamers from the CRP-MNPs, and the eluted aptamers in the supernatant were collected by using the magnetic stand. Then the collected supernatant was subjected to a negative selection which will be described below. After the negative selection, the final supernatant was denoted as >27K. The above mentioned magnetic screening by applying oscillation magnetic fields is referred as a positive selection. Since the target-streptavidin-magnetic nanoparticles were used during the positive selection, the obtained aptamers may possibly be selected from those bound to SA-MNPs instead of the target. Therefore, each collected supernatant was subjected to a negative selection which was performed by using SA-MNPs to eliminate any possibility of aptamers that could bind to SA-MNPs. The negative selection may be performed before or after the positive selection. For the negative selection, SA-MNPs, obtained from washing 5 µl of SA-MNP reagent, were added to the collected supernatant and incubated for 30 minutes at room temperature. Then the collected supernatant was subjected to a magnetic separation by using the magnetic stand to remove aptamer-streptavidin-magnetic nanoparticles and the supernatant was collected. It must be noted that SA-MMPs can also be used for the negative selection. The supernatant obtained after the negative selection from various rounds of magnetic-assisted screening with different field frequencies were named as the corresponding frequency. The collected supernatants were precipitated with 1 ml of 100% cold alcohol for further analysis, such as PCR amplification, cloning and sequencing.

Characterization of Aptamers Selected by Using RO-MARAS with Magnetic Nanoparticles

The aptamers obtained from the magnetic-assisted screening (positive selection and negative selection) of the various rotating magnetic field frequencies were named as, 20N RO F2(-1, -2), 20N RO F20(-4, -5), 20N RO F200(-1, -3), 20N RO F2K(-1, -4), 20N RO F20K(-1, -2), 20N RO F27K(-4, -6) and 20N RO>27K(-1, -5) and the sequencing results of the selected 20N aptamers were listed in the Table 1.

**Table 1**

| Aptamer | 5'-sequence-3' | |
|---|---|---|
| 20N RO F2-1 | AGACCTTTGAAGGCCCCAAG | (SEQ ID NO.4) |
| 20N RO F2-2 | TTGGTTTGTAACATTGATGG | (SEQ ID NO.5) |
| 20N RO F20-4 | AGTTAGTTTCAGTGGGTCCC | (SEQ ID NO.6) |
| 20N RO F20-5 | ACCGTGTTGTCTGAGGTCTC | (SEQ ID NO.7) |
| 20N RO F200-1 | TCGGTGTGCCACTTTCTCAA | (SEQ ID NO.8) |
| 20N RO F200-3 | TTTTAGTTGTGCCTGGTGCG | (SEQ ID NO.9) |
| 20N RO F2K-1 | GGTGTGCGTTATTCTGTGGC | (SEQ ID NO.10) |
| 20N RO F2K-4 | CTCTTGGACAACTGGATTAG | (SEQ ID NO.11) |
| 20N RO F20K-1 | ACCCTACGTACGTTCTCATG | (SEQ ID NO. 12) |
| 20N RO F20K-2 | ATATAATTTTGCTCTTTATT | (SEQ ID NO. 13) |
| 20N RO F27K-4 | GCCCCCGAAGTTGCTTAGTC | (SEQ ID NO. 14) |
| 20N RO F27K-6 | CACATTAGGTGTGATAAGGC | (SEQ ID NO. 15) |
| 20N RO >27K-1 | TCTGTAATTTATAGTTCCAT | (SEQ ID NO.16) |
| 20N RO >27K-5 | TTTTTCGCTGGTGTTCGACC | (SEQ ID NO. 17) |

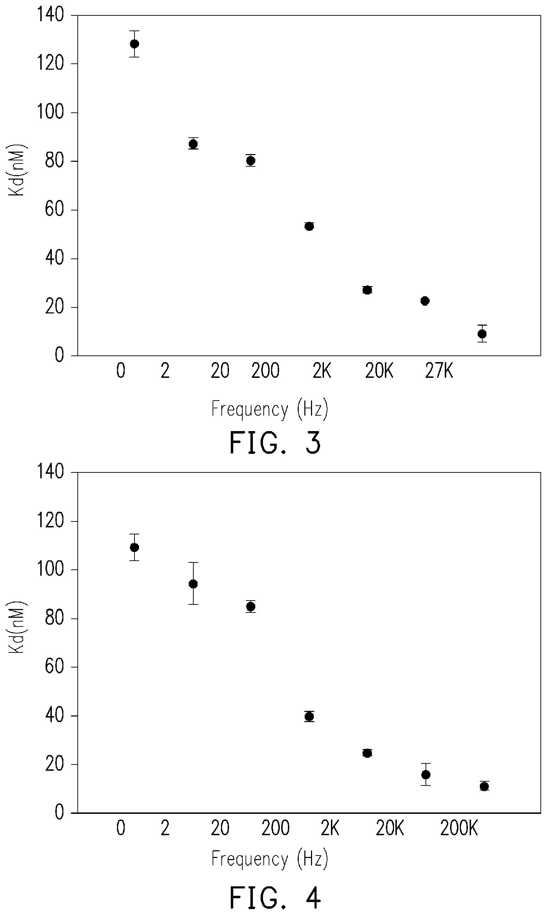

The dissociation constants (Kds) were calculated by q-PCR and non-linear fitting curve. Figure 3 shows the relationship of the magnetic field frequency used in RO-MARAS and dissociation constants (Kd) of the selected 20N aptamers screened by RO-MARAS according to one embodiment of the present invention. The results of Fig. 3 show that the dissociation constant (Kd) of the selected 20N aptamers screened by RO-MARAS decreases as the magnetic field frequency of RO-MARAS increases. The lowest Kd value of 20N aptamer screened by RO-MARAS at a constant field strength of 14 gauss with a series of frequency was 6.26 nM for the 20N RO>27K-1 aptamer. In other words, the aptamers having higher binding affinity can be screened by increasing the field frequency of RO-MARAS. This confirms the competitive mechanism and indicates that the affinity of selected aptamer to the target can be enhanced by increasing the frequency of the driving field. This also demonstrates that the affinity of the screened aptamers to the target molecule by RO-MARAS is dependent on the applied rotating magnetic field frequency. In order to optimize the selection parameters of the MARAS at higher field frequency and field strength, the effects of the applied alternating magnetic field (AC-MARAS), including field strength and frequency, on the affinity of the selected aptamers were investigated. The preparation of bound mixtures of 20N randomized oligonucleotide sequence library with CRP-MNPs was as described above. In a field frequency dependent analysis, an AC magnetic field with a constant magnetic field strength (25 gauss) and varying in the magnetic field frequency (e.g.: 2 Hz, 20 Hz, 200 Hz, 2 KHz, 20 KHz and 200 KHz) was applied via the setup of Fig. 1B. First, the bound mixture was subjected to an AC magnetic field of 25 gauss and 2 Hz for 10 minutes. During the application of magnetic field, the mixture was stirred every 2.5 minutes by pipetting. After one round of the magnetic-assisted screening, the magnetic separation was performed to collect the supernatant and the bound mixture was re-suspended in 100 µl BD buffer. The mixture was sequentially subjected to another AC magnetic field with higher frequency (such as 20 Hz). The above procedure was repeated until an AC magnetic field with the final frequency (200 KHz) was applied.

After the application of the magnetic field of the final frequency, a magnetic separation was performed to separate supernatant and the bound mixture was retained. Finally, 100 µl BD buffer was added to re-suspend the retained bound mixture, and then was heated to 95°C for 5 minutes for elution. The eluted aptamers in the supernatant were separated by the magnetic stand and the collected supernatant was subjected to the negative selection as described above. The final supernatant going through the negative selection to eliminate any aptamer that could bind to SA-MNPs was denoted as >200K. Also, a negative selection was performed for each collected supernatant obtained from applying AC magnetic field with different frequency to remove aptamer-streptavidin-magnetic nanoparticles and the supernatant was collected. The negative selection may be performed before or after the positive selection in AC-MARAS. The supernatant, after the negative selection, obtained from various rounds of magnetic-assisted screening with different field frequencies were named as the corresponding frequency. All the collected supernatants were precipitated with 1 ml of 100% cold alcohol for further analysis, such as cloning, PCR amplification and sequencing.

### Characterization of Aptamers Selected by Using AC-MARAS with Magnetic Nanoparticles

Based on the 20N randomized nucleotide sequence library, under the alternating magnetic field (AC) and at the field frequency of 2 Hz, 20 Hz, 200 Hz, 2000 Hz, 20 KHz and 200 KHz, these collected unbound nucleotide sequences were denoted as 20N AC F2(-2, -4), F20(-2, -4), F200(-1, -2), F2K(-1, -4), F20K(-1, -3), F200K(-1, -2), >200K(-1, -2) sequences with individual reference numbers (as listed in Table 2).

**Table 2**

| Aptamer | 5'-sequence-3' | |
|---|---|---|
| 20N AC F2-2 | ATTGTTCGTTATTGCGTTCG | (SEQ ID NO.18) |
| 20N AC F2-4 | ATACTCGCCGCTTTTGAATT | (SEQ ID NO.19) |
| 20N AC F20-2 | TATGCGGGTGTTTGCGACTG | (SEQ ID NO.20) |
| 20N AC F20-4 | CTTTCTTTCTAGGTGTGTG | (SEQ ID NO.21) |
| 20N AC F200-1 | TGACTTACATGTACTACTTT | (SEQ ID NO.22) |
| 20N AC F200-2 | TGTCAGTTTGATCTGTAATT | (SEQ ID NO.23) |
| 20N AC F2K-1 | CTCCTTTTGATTTGCGGGTA | (SEQ ID NO.24) |
| 20N AC F2K-4 | TTGGCTTTAACTATATGGAG | (SEQ ID NO.25) |
| 20N AC F20K-1 | TCTACGCGATATTCTCCCCC | (SEQ ID NO.26) |
| 20N AC F20K-3 | TCTTCCGCCGTACCGTTCCC | (SEQ ID NO.27) |
| 20N AC F200K-1 | GCATTGTATCACAGGTACTG | (SEQ ID NO.28) |
| 20N AC F200K-2 | TTGTCTCTGGGGATCTAAAC | (SEQ ID NO.29) |
| 20N AC >200K-1 | TCCTGTTGATGTGTGTTATATG | (SEQ ID NO.30) |
| 20N AC >200K-2 | AATTACAAATTTGGACTGTT | (SEQ ID NO.31) |

Figure 4 shows the relationship of the magnetic field frequency used in AC-MARAS and dissociation constants (Kd) of the selected 20N aptamers screened by AC-MARAS according to one embodiment of the present invention. The results of Fig. 4 show that the dissociation constant (Kd) of the selected 20N aptamers screened by AC-MARAS decreases as the magnetic field frequency of AC-MARAS increases. The lowest Kd value of the 20N aptamers screened by AC-MARAS at 25 gauss was 8.35 nM for the 20N AC >200K-2 aptamer. The corresponding Kd values and the curves of the Kd values versus the field frequency obtained through AC-MARAS or through RO-MARAS are similar. Based on the results of Fig. 3 or 4, the Kds of the selected aptamers is frequency-dependent manner for either RO-MARAS or AC-MARAS.

According above results, the binding affinity of the aptamer(s) selected by MARAS (including AC-MARAS and RO-MARAS) is highly dependent on the applied magnetic field frequency of MARAS. To optimize the magnetic field condition of the MARAS, the higher magnetic field strength was used in subsequent experiments.

For a field strength dependent analysis, AC-MARAS using an AC magnetic field of a fixed frequency (100 KHz) and varying in the field strength (e.g.: 12.5 gauss, 25 gauss, 50 gauss, 100 gauss, 200 gauss and 400 gauss) was performed with the setting of Fig. 1B. The experimental procedures were similar to the procedures of frequency dependent analysis. In addition, the collected supernatants were denoted using a similar designation scheme as described above. The series of the supernatants after the negative selection were collected and all the collected supernatants were precipitated with 1 ml of 100% cold alcohol for further PCR amplification, cloning and sequencing.

### Characterization of Selected Aptamers from High Magnetic Field Strength MARAS (HAC-MARAS)

Based on the 20N randomized nucleotide sequence library, under the alternating magnetic field (AC), at the fixed field frequency of 100 KHz and at the high field strength of 12.5 gauss (12.5g), 25 gauss, 50 gauss, 100 gauss, 200 gauss and 400 gauss, these collected aptamer sequences were denoted as, 20N HAC 12.5g(-1, -2), 20N HAC 25g(-1, -2), 20N HAC 50g(-2, -9), 20N HAC 100g(-1, -2), 20N HAC 200g(-2, -3), 20N HAC 400g(-1, -5) and 20N HAC >400g(-1, -4) and the sequencing results of the aptamers were listed in Table 3.

**Table 3**

| Aptamer | 5'-sequence-3' | |
|---|---|---|
| 20N HAC 12.5g-1 | TTGCATAGTTCGGTTCTATG | (SEQ ID NO. 32) |
| 20N HAC 12.5g-2 | TGTATGGTGGTTCCATCTTC | (SEQ ID NO. 33) |
| 20N HAC 25g-1 | TATGTATGGAATCACAATCC | (SEQ ID NO. 34) |
| 20N HAC 25g-2 | TTGCGGGATGAAGTGTCTAA | (SEQ ID NO. 35) |
| 20N HAC 50g-2 | TTGGTATTCCTCGCGTTTTT | (SEQ ID NO. 36) |
| 20N HAC 50g-9 | GCGTTTGGCATCCCTCGGTC | (SEQ ID NO. 37) |
| 20N HAC 100g-1 | CTGTGGTTGGCGCTTGGCAT | (SEQ ID NO. 38) |
| 20N HAC 100g-2 | TGGACTTCATTTTAAGTATG | (SEQ ID NO. 39) |
| 20N HAC 200g-2 | GTTTACTGTTTTATTGCGCT | (SEQ ID NO. 40) |
| 20N HAC 200g-3 | TATGATAAGGTGATAACATG | (SEQ ID NO. 41) |
| 20N HAC 400g-1 | TTGGCACAAAGCGGTGTTAC | (SEQ ID NO. 42) |
| 20N HAC 400g-5 | GTGTCGTTCGGTGCATCATG | (SEQ ID NO. 43) |
| 20N HAC >400g-1 | TGCTATTCAGGTGGACTATG | (SEQ ID NO. 44) |
| 20N HAC >400g-4 | TGGCAACTTTTGAACGCGTT | (SEQ ID NO. 45) |

| | | |
|---|---|---|
| HAC: High Magnetic Field Strength | | |

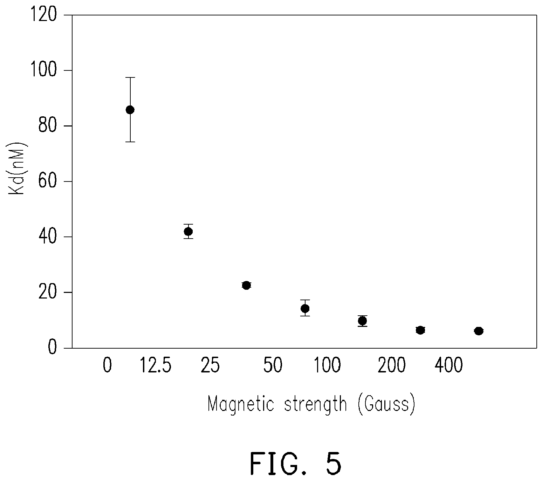

The lowest Kd value of the 20N aptamers screened by HAC-MARAS at a fixed field frequency of 100 KHz was 5.67 nM for the 20N HAC 400g-1 aptamer. Figure 5 shows the relationship of the magnetic field strength used in HAC-MARAS and dissociation constants (Kd) of the selected 20N aptamers screened by HAC-MARAS according to one embodiment of the present invention. The curve of Fig. 5 demonstrates that the Kd value of the selected 20N aptamers decreases as the applied magnetic field strength of HAC-MARAS increases. Hence, the higher affinity aptamer to the target can be obtained by enhancing the competitive mechanism resulted from the increase of the field strength of the applied AC magnetic field. The enhancement is due to the larger driving torque resulted from a higher magnetic field strength and the larger instantaneous tangential velocity when the magnetic particle rotates or oscillates under an oscillation magnetic field with higher strength. It also can be inferred that the same enhancement of the competitive mechanism can be achieved for RO-MARAS by increasing the field strength of the rotating magnetic field.

Hence, the binding affinity of the aptamers screened by MARAS is dependent on either the strength or the frequency of the applied magnetic field. Furthermore, an aptamer to a target molecule with a desired range of dissociation constant can be selected by properly choosing a lower-cutoff and an upper-cutoff frequencies and/or strength of applied magnetic fields of MARAS protocol(s).

### One-Shot MARAS (OS-MARAS) Procedure

To further demonstrate the powerfulness of MARAS protocols for the selection of aptamers which have high binding affinity to the target molecule, one-shot MARAS (OS-MARAS) was performed using the setting of Fig. 1A for RO-MARAS. In the one-shot MARAS experiments, three types of randomized oligonucleotide sequences (60N, 40N and 20N) having the middle sections of different lengths are used as the sequence libraries to concurrently evaluate the influences of the sequence lengths on the affinity of selected aptamers to the target molecule. The one-shot RO-MARAS (OS-RO-MARAS) experiment was performed at 14 gauss and 20 KHz. The bound mixtures of 60N, 40N and 20N libraries with CRP-MNPs were prepared as described above. Each bound mixture was subjected to a rotating magnetic field of 14 gauss and 20 KHz for 10 minutes and stirred every 2.5 minutes by pipetting. After one round of the magnetic-assisted screening, the supernatant was removed by using the magnetic stand and the retained bound mixture was re-suspended with 100 µl BD buffer. Afterward, the retained bound mixture was heated to 95°C for 5 minutes for elution. The eluted aptamers in the supernatant were separated by the magnetic stand and the collected supernatant was subjected to the negative selection as described above. The negative selection was performed to eliminate the possible SA-MNP bound aptamers. Another round of magnetic separation was performed to collect the supernatant which was then precipitated with 1 ml of 100% cold alcohol for further analysis. Two aptamers were selected from each experiment for sequencing and binding affinity calculation. Also, one-shot AC-MARAS (OS-AC-MARAS) with the high magnetic field using the setting of Fig. 1B of AC-MARAS was performed following the similar procedure described above, using an AC magnetic field at 100 gauss and 150 KHz.

The selected 60N, 40N, and 20N aptamers screened by one-shot RO-MARAS (OS-RO-MARAS) were named as, OS-RO-60N>20K(-1, -2), OS-RO-40N>20K(-1, -2), and OS-RO-20N>20K(-3, -6) and the sequencing results were listed in Table 4. For the OS-RO-MARAS experiments, the screened 20N aptamers correspond to the 20N aptamers named F27K and >27K selected via RO-MARAS with successive rounds of the magnetic-assisted screening.

**Table 4**

| Aptamer | 5'-sequence-3' | |
|---|---|---|
| OS-RO-60N >20K-1 | GCCTTTGAATTTTATTTCAACACGTCTTTTATTGTTGTTAAGTTCTTGATAGTACTTTAA (SEQ ID NO. 46) | |
| OS-RO-60N >20K-2 | TTCGCTAAGTGTTGGACTTTGTGAAGCTTCCCAGATATACGTTTGTGTGCTTGGAACTTC (SEQ ID NO. 47) | |
| OS-RO-40N >20K-1 | TTGTTTGTTTTGCTTGTTACTGCTTTCTCTTCTCTTTTCA | (SEQ ID NO. 48) |
| OS-RO-40N >20K-2 | TTTATGTGTGTTTTGTTTATTGCTACTGTGTCTATCTATT | (SEQ ID NO. 49) |
| OS-RO-20N >20K-3 | TACAGTTTAGTGAGTGCAAG | (SEQ ID NO. 50) |
| OS-RO-20N >20K-6 | CTATGTGAGTATTTCGCCAC | (SEQ ID NO. 51) |

The binding affinities of 60N, 40N and 20N aptamers were calculated by q-PCR and non-linear fitting curve, and the average values of the dissociation constants Kd of the selected aptamers from one-shot RO-MARAS experiments were shown in Table 5.

**Table 5**

| Aptamers | Dissociation constant Kd (nM) |
|---|---|
| OS-RO-60N >20KHz | 12.42±0.90 (13.06, 11.78) |
| OS-RO-40N >20KHz | 13.26±1.80 (11.99, 14.54) |
| OS-RO-20N >20KHz | 12.19±0.31 (11.97, 12.41) |

The results indicated that the Kd values of the aptamers screened by OS-RO-MARAS were consistent with previous results, i.e. the Kd values of the aptamers. For example, the aptamer OS-RO-20N>20 KHz with Kd of 12.19±0.31 nM vs. the aptamer 20N RO F27K-6 with Kd of 22.53 nM and the aptamer 20N RO >27K-1 with Kd of 6.26 nM in Fig. 3.

For one-shot AC-MARAS performed at 100 gauss and of the frequency of 150 KHz, the selected 60N, 40N, and 20N aptamers screened by OS-AC-MARAS were named as, OS-AC-60N(-1, -2), OS-AC-40N(-6, -7), and OS-AC-20N(-1, -2), and the sequencing results of the selected aptamers were shown in Table 6.

**Table 6**

| Aptamer | 5'-sequence-3' | |
|---|---|---|
| OS-AC-60N-1 | TCTGTTGGATAATTCTTGCTTAATGTCGAATACTCTTACACATCATTTCTTTCATGCATC (SEQ ID NO.52) | |
| OS-AC-60N-2 | TTATTTACTGCTCTTGTGTCACGTTGTTTAGAGCTGTACTTGCTTTAATATCTGGTTTAA (SEQ ID NO. 53) | |
| OS-AC-40N-6 | TAAATTTTTGTATTAGTTTTTGCATTAGTTCTGCCATACT | (SEQ ID NO. 54) |
| OS-AC-40N-7 | TTACTTGTTTCTTAACTCAATGAGTTGTTTTGTCATGTCG | (SEQ ID NO. 55) |
| OS-AC-20N-1 | GCATGAACCACTAAGTGGGT | (SEQ ID NO. 56) |
| OS-AC-20N-2 | GCCTGATTCATTGTCGGCGC | (SEQ ID NO. 57) |

The average values of Kd of the selected 60N, 40N and 20N aptamers from one-shot AC-MARAS experiments were shown in Table 7.

**Table 7**

| Aptamers | Dissociation constant Kd (nM) |
|---|---|
| OS-AC-60N | 12.52±0.07 (12.47, 12.57) |
| OS-AC-40N | 12.32±0.36 (12.58, 12.07) |
| OS-AC-20N | 11.10±0.87 (11.72, 10.49) |

When comparing the results of dissociation constants of aptamers selected from different lengths (60N, 40N, and 20N) of randomized oligonucleotide sequences, it demonstrates that the binding affinity of the selected aptamers is independent of the length of the randomized oligonucleotide sequences for MARAS protocols.

In addition, as mentioned before, the negative selection can be performed priors to the positive selection, which is called a reversed MARAS protocol. For the reversed RO-MARAS experiment, 20N randomized oligonucleotide sequence was used as the initial library. One nanomole of 20N randomized oligonucleotide sequence library was subjected to the negative selection first and the magnetic separation was performed to separate the supernatant from the bound mixture by using a magnetic stand. The preparation of the library and the procedure of the negative selection were as described above. The collected supernatant going through the negative selection was served as a reduced library. The reduced library was incubated with CRP-MNPs as described above. The bound mixture was subjected to a rotating magnetic field of 14 gauss and 20 KHz for 10 minutes and stirred every 2.5 minutes by pipetting. After one round of the magnetic-assisted screening, the supernatant was removed and the retained bound mixture was re-suspended with 100 µl BD buffer. Afterward, the mixture was heated to 95°C for 5 minutes for elution and another round of the magnetic separation was performed to collect the supernatant which was then precipitated with 1 ml of 100% cold alcohol for further analysis. The reversed AC-MARAS experiment was performed at an alternating magnetic field of 150 KHz and 100 Gauss. The procedures of reversed AC-MARAS were similar to those of the reversed RO-MARAS.

Table 8 shows the comparison of the dissociation constants Kd of the aptamers screened by MARAS and by reversed MARAS.

**Table 8**

| Aptamers | Dissociation constant Kd (nM) |
|---|---|
| 20N RO-MARAS | 12.19±0.31 (11.97, 12.41) |
| 20N RO-MARAS (reversed) | 15.15±3.31 (17.50, 12.81) |
| 20N AC-MARAS | 11.10±0.87 (11.72, 10.49) |
| 20N AC-MARAS (reversed) | 11.35±1.47 (12.39, 10.31) |

The results of Table 8 show that the dissociation constants of the selected aptamers from MARAS and reversed MARAS were similar and comparable. Therefore, the performing sequence of the positive selection and the negative selection (i.e. positive selection first or negative selection first) will not alter the quality (binding affinity to the target in terms of the dissociation constant) of the selected aptamers.

In order to validate the magnetic particles used for MARAS are not limited to nanometer size, Mi-MARAS was performed by using magnetic microparticles (Mi). CRP-MNPs were substituted by CRP-MMPs during the positive and negative selections. The preparation of the bound mixture for aptamer-CRP-MMPs was the same as that for aptamer-CRP-MNPs, except for using CRP-MMPs. The mixture was subjected to an AC magnetic field for 10 minutes and stirred every 2.5 minutes by pipetting to avoid the agglomeration and precipitation of micro magnetic particles. An AC magnetic field with frequency of 100 KHz and strength of 400 gauss was used for the one-shot Mi-MARAS protocol. After the positive selection, the negative selection was performed to eliminate the possible SA-MMP bound aptamers by using SA-MMPs, obtained from washing 5 µl of SA-MMP reagent. The procedure for the negative selection was the same as that described before, except for using SA-MMPs. It must be noted the SA-MNPs can also be used for the negative selection. The detail processes were as described in the above sections. The selected 20N aptamers screened by one-shot Mi-MARAS were named as, OS-Mi-20N-1 and OS-Mi-20N-2 and the sequencing results of the selected aptamers were shown in Table 9.

**Table 9**

| Aptamer | 5'-sequence-3' | |
|---|---|---|
| OS-Mi-20N-1 | GCCTAAAGCTGGATTCCCTG | (SEQ ID NO. 58) |
| OS-Mi-20N-2 | GCCTGATTCATTGTCGGCGC | (SEQ ID NO. 59) |

The average value of Kd of the aptamers selected from one-shot Mi-MARAS experiments were 0.825±0.71 nM (OS-Mi-20N-1 of Kd 0.33 nM and OS-Mi-20N-2 of Kd 1.33 nM). It demonstrates that the magnetic particles with a larger size can enhance the competitive mechanism by increasing the magnetic torque and the dissipative torque acting on the magnetic particles. The enhancement is due to the higher driving torque resulted from a larger dipole moment of magnetic particle and the higher dissipative torque resulted from a higher tangential velocity of magnetic particle as it rotates or oscillates in an aqueous solution.

In summary, the aptamers selected through the MARAS processes can interact with targets. Also, the dissociation constants of the aptamers selected via MARAS are magnetic field dependent, of which the Kd value decreases as the field frequency and field strength increase. In addition, the dissociation constants of the single strand aptamers binding with target may be at the level of a few nanomolar (nM) by applying a rotating or alternating magnetic field of a suitable frequency and strength under MARAS protocols. Furthermore, if the biofunctionalized magnetic micro-particles were used, instead of magnetic nanoparticles, in the MARAS protocols, the dissociation constants of the selected aptamers could even reach a picomolar (pM) level. The binding affinity of the single strand aptamers selected by AC-MARAS was similar to that of the aptamers selected by RO-MARAS under the same field conditions. Moreover, one or more aptamers specific to and having a predetermined binding affinity to a target molecule (i.e. having the dissociation constant within a desirable range) can be selected by properly choosing a lower-cutoff and an upper-cutoff field frequencies and/or field strengths of the applied magnetic field of the MARAS protocols. As compared to the traditional ss-SELEX processes requiring several weeks to several months, the MARAS protocols described in this study can be completed within less than an hour and consume fewer experimental supplies and labor work.

## Claims

1. An aptamer selection protocol, comprising:
a) providing a sample comprising at least a random oligonucleotide library having a plurality of oligonucleotides and a plurality of magnetic nanoparticles or micro-particles having target molecules joined thereon, wherein a first portion of the plurality of oligonucleotides is bound to the plurality of magnetic nanoparticles or micro-particles having the target molecules joined thereon;
b) performing a first round of magnetic separation by using a magnetic stand to collect the first portion of the plurality of oligonucleotides bound to the plurality of magnetic nanoparticles or micro-particles having the target molecules joined thereon;
c) dispersing the first portion of the plurality of oligonucleotides bound to the plurality of magnetic nanoparticles or micro-particles having the target molecules joined thereon in a binding buffer;
d) performing a magnetic-assisted screening by applying an oscillation magnetic field to the first portion of the plurality of oligonucleotides bound to the plurality of magnetic nanoparticles or micro-particles having the target molecules joined thereon, so that a portion of the first portion of the plurality of oligonucleotides is detached from the plurality of magnetic nanoparticles or micro-particles having the target molecules joined thereon;
e) performing a second round of magnetic separation by using the magnetic stand to collect a second portion of the first portion of the plurality of oligonucleotides that remained bound to the plurality of magnetic nanoparticles or micro-particles having the target molecules joined thereon after step d), so that the portion of the first portion of the plurality of oligonucleotides detached from the plurality of magnetic nanoparticles or micro-particles having the target molecules jointed thereon is removed;
f) dispersing the second portion of the first portion of the plurality of oligonucleotides that remained bound to the plurality of magnetic nanoparticles or micro-particles having the target molecules joined thereon after step d) in a binding buffer;
g) eluting the second portion of the first portion of the plurality of oliganuclcotides that remained bound to the plurality of magnetic nanoparticles or micro-particles having the target molecules joined thereon after step d) from the plurality of magnetic nanoparticles or micro-particles having the target molecules joined thereon;
h) performing a third round of magnetic separation by using the magnetic stand to remove the plurality of magnetic nanoparticles or micro-particles having the target molecules joined thereon from the eluted second portion of the first portion of the plurality of oligonucleotides; and
i) performing a negative selection to the eluted second portion of the first portion of the plurality of oligonucleotides to select aptamers specific to the target molecules, wherein the aptamers specific to the target molecules are not bound to the plurality of magnetic nanoparticles or micro-particles without having the target molecules joined thereon,
wherein the target molecules are joined to the plurality of magnetic nanoparticles or micro-particles through conjugation pairs, the conjugation pairs include first components attached to the plurality of magnetic nanoparticles or micro-particles and second components attached to the target molecules, and
wherein step i) performing the negative selection comprises:
adding a plurality of magnetic nanoparticles or micro-particles having the first components attached thereon to the eluted second portion of the first portion of the plurality of oligonucleotides;
incubating the plurality of magnetic nanoparticles or micro-particles having the first components attached thereon with the eluted second portion of the first portion of the plurality of oligonucleotides; and
performing a fourth round of magnetic separation to obtain the aptamers specific to the target molecules by removing the plurality of oligonucleotides bound to the plurality of magnetic nanoparticles or micro-particles having the first components attached thereon.

2. The aptamer selection protocol as claimed in claim 1, wherein the oscillation magnetic field is a rotating magnetic field.

3. The aptamer selection protocol as claimed in claim 2, wherein the frequency of the rotating magnetic field is from 1 Hz to 300 KHz.

4. The aptamer selection protocol as claimed in claim 2, wherein the strength of the rotating magnetic field is from 5 gauss to 1000 gauss.

5. The aptamer selection protocol as claimed in claim 1, wherein the oscillation magnetic field is an alternating magnetic field.

6. The aptamer selection protocol as claimed in claim 5, wherein the frequency of the alternating magnetic field is from 1 Hz to 300 KHz.

7. The aptamer selection protocol as claimed in claim 5, wherein the strength of the alternating magnetic field is from 5 gauss to 1000 gauss.

8. The aptamer selection protocol as claimed in claim 1, wherein the size of the magnetic nanoparticle is from 5 nm to 500 nm.

9. The aptamer selection protocol as claimed in claim 1, wherein the size of the magnetic micro-particle is from 0.50 µm to 50 µm.

10. An aptamer selection protocol, comprising:
a) providing a sample comprising at least a random oligonucleotide library having a plurality of oligonucleotides and diluting with a binding buffer;
b) performing a negative selection to the sample by incubating with a plurality of first magnetic nanoparticles or micro-particles without having target molecules joined thereon to isolate a first portion of the plurality of oligonucleotides not bound to the plurality of first magnetic nanoparticles or micro-particles without having the target molecules joined thereon;
c) performing a first round of magnetic separation to remove the plurality of oligonucleotides bound to the plurality of first magnetic nanoparticles or micro-particles without having the target molecules joined thereon from the first portion of the plurality of oligonucleotides;
d) incubating the first portion of the plurality of oligonucleotides with a plurality of second magnetic nanoparticles or micro-particles having the target molecules joined thereon, wherein a second portion of the first portion of the plurality of oligonucleotides is bound to the plurality of magnetic nanoparticles or micro-particles having the target molecules joined thereon;
e) performing a second round of magnetic separation to collect the second portion of the first portion of the plurality of oligonucleotides bound to the plurality of second magnetic nanoparticles or micro-particles having the target molecules joined thereon;
f) dispersing the second portion of the first portion of the plurality of oligonucleotides bound to the plurality of second magnetic nanoparticles or micro-particles having the target molecules joined thereon in a binding buffer;
g) performing a magnetic-assisted screening by applying an oscillation magnetic field to the second portion of the first portion of the plurality of oligonucleotides bound to the plurality of second magnetic nanoparticles or micro-particles having the target molecules joined thereon, so that a portion of the second portion of the first portion of the plurality of oligonucleotides is detached from the plurality of second magnetic nanoparticles or micro-particles having the target molecules joined thereon;
h) performing a third round of magnetic separation by using a magnetic stand to collect a third portion of the second portion of the first portion of the plurality of oligonucleotides that remained bound to the plurality of second magnetic nanoparticles or micro-particles having the target molecules joined thereon after step g), so that the portion of the second portion of the first portion of the plurality of oligonucleotides detached from the plurality of second magnetic nanoparticles or micro-particles having the target molecules joined thereon is removed;
i) dispersing the third portion of the second portion of the first portion of the plurality of oligonucleotides that remained bound to the plurality of second magnetic nanoparticles or micro-particles having the target molecules joined thereon after step g) with ddH₂O;
j) eluting the third portion of the second portion of the first portion of the plurality of oligonucleotides that remained bound to the plurality of second magnetic nanoparticles or micro-particles having the target molecules joined thereon after step g) from the plurality of second magnetic nanoparticles or micro-particles having the target molecules joined thereon; and
k) performing a fourth round of magnetic separation to remove the plurality of second magnetic nanoparticles or micro-particles having the target molecules joined thereon from the eluted third portion of the second portion of the first portion of the plurality of oligonucleotides and to obtain aptamers specific to the target molecules,
wherein the target molecules are joined to the plurality of second magnetic nanoparticles or micro-particles through conjugation pairs, and the conjugation pairs include first components attached to the plurality of second magnetic nanoparticles or micro-particles and second components attached to the target molecules, and the plurality of the first magnetic nanoparticles or micro-particles has the first components joined thereon.

11. The aptamer selection protocol as claimed in claim 10, wherein the oscillation magnetic field is a rotating magnetic field.

12. The aptamer selection protocol as claimed in claim 11, wherein the frequency of the rotating magnetic field is from 1 Hz to 300 KHz.

13. The aptamer selection protocol as claimed in claim 11, wherein the strength of the rotating magnetic field is from 5 gauss to 1000 gauss.

14. The aptamer selection protocol as claimed in claim 10, wherein the oscillation magnetic field is an alternating magnetic field.

15. The aptamer selection protocol as claimed in claim 14, wherein the frequency of the alternating magnetic field is from 1 Hz to 300 KHz.

16. The aptamer selection protocol as claimed in claim 14, wherein the strength of the alternating magnetic field is from 5 gauss to 1000 gauss.

17. The aptamer selection protocol as claimed in claim 10, wherein the size of the first or second magnetic nanoparticle is from 5 nm to 500 nm.

18. The aptamer selection protocol as claimed in claim 10, wherein the size of the first or second magnetic micro-particle is from 0.50 µm to 50 µm.

19. An aptamer selection protocol with a desired affinity range of the selected aptamer to the target, comprising:
a) providing a sample comprising at least a random oligonucleotide library having a plurality of oligonucleotides and a plurality of magnetic nanoparticles or micro-particles having target molecules joined thereon, wherein a first portion of the plurality of oligonucleotides is bound to the plurality of magnetic nanoparticles or micro-particles having the target molecules joined thereon;
b) performing a first round of magnetic separation by using a magnetic stand to collect the first portion of the plurality of oligonucleotides bound to the plurality of magnetic nanoparticles or micro-particles having the target molecules joined thereon;
c) dispersing the first portion of the plurality of oligonucleotides bound to the plurality of magnetic nanoparticles or micro-particles having the target molecules joined thereon in a binding buffer;
d) performing a first magnetic-assisted screening by applying a first oscillation magnetic field to the first portion of the plurality of oligonucleotides bound to the plurality of magnetic nanoparticles or micro-particles having the target molecules joined thereon, so that a portion of the first portion of the plurality of oligonucleotides is detached from the plurality of magnetic nanoparticles or micro-particles having the target molecules joined thereon;
e) performing a second round of magnetic separation by using the magnetic stand to collect a second portion of the first portion of the plurality of oligonucleotides that remained bound to the plurality of magnetic nanoparticles or micro-particles having the target molecules joined thereon after step d), so that the portion of the first portion of the plurality of oligonucleotides detached from the plurality of magnetic nanoparticles or micro-particles having the target molecules joined thereon is removed:
f) dispersing the second portion of the first portion of the plurality of oligonucleotides that remained bound to the plurality of magnetic nanoparticles or micro-particles having the target molecules joined thereon after step d) in a binding buffer;
g) performing a second magnetic-assisted screening by applying a second oscillation magnetic field having a field frequency and/or field strength higher than those of the first oscillation magnetic field to the second portion of the first portion of the plurality of oligonucleotides that remained bound to the plurality of magnetic nanoparticles or micro-particles having the target molecules joined thereon after step d), so that a portion of the second portion of the first portion of the plurality of oligonucleotides that remained bound in step d) is detached from the plurality of magnetic nanoparticles or micro-particles having the target molecules joined thereon;
h) performing a third round of magnetic separation by using the magnetic stand to collect a third portion or the second portion of the first portion of the plurality of oligonucleotides detached from the plurality of magnetic nanoparticles or micro-particles having the target molecules joined thereon; and
i) performing a negative selection to the third portion of the second portion of the first portion of the plurality of oligonucleotides to select aptamers specific to the target molecules, wherein the aptamers specific to the target molecules with the desirable affinity are not bound to the plurality of magnetic nanoparticles or micro-particles without having the target molecules joined thereon,
wherein the target molecules are joined to the plurality of magnetic nanoparticles or micro-particles through conjugation pairs, and the conjugation pairs include first components attached to the plurality of magnetic nanoparticles or micro-particles and second components attached to the target molecules, and
wherein step i) performing the negative selection comprises:
adding a plurality of magnetic nanoparticles or micro-particles having the first components attached thereon;
incubating the plurality of magnetic nanoparticles or micro-particles having the first components attached thereon with the third portion of the second portion of the first portion of the plurality of oligonucleotides; and
performing a fourth round of magnetic separation to obtain the aptamers specific to the target molecules by removing the plurality of oligonucleotides bound to the plurality of magnetic nanoparticles or micro-particles having the first components attached thereon.

20. The aptamer selection protocol as claimed in claim 19, wherein the first and the second oscillation magnetic fields are rotating magnetic fields.

21. The aptamer selection protocol as claimed in claim 20, wherein the field frequency of the rotating magnetic fields is from 1 Hz to 300 KHz.

22. The aptamer selection protocol as claimed in claim 20, wherein the field strength of the rotating magnetic fields is from 5 gauss to 1000 gauss.

23. The aptamer selection protocol as claimed in claim 19, wherein the first and the second oscillation magnetic fields are alternating magnetic fields.

24. The aptamer selection protocol as claimed in claim 23, wherein the field frequency of the alternating magnetic fields is from 1 Hz to 300 KHz.

25. The aptamer selection protocol as claimed in claim 23, wherein the field strength of the alternating magnetic fields is from 5 gauss to 1000 gauss.

26. The aptamer selection protocol as claimed in claim 19, wherein the size of the magnetic nanoparticle is from 5 nm to 500 nm.

27. The aptamer selection protocol as claimed in claim 19, wherein the size of the magnetic micro-particle is from 0.50 µm to 50 µm.

## Patentansprüche

1. Aptamerselektionsprotokoll, umfassend:
a) das Bereitstellen einer Probe umfassend mindestens eine Zufalls-Oligonucleotid-Bibliothek, die mehrere Oligonucleotide und mehrere magnetische Nanopartikel oder Mikropartikel aufweist, die daran angefügte Targetmoleküle aufweisen, wobei ein erster Teil der mehreren Oligonucleotide an die mehreren magnetischen Nanopartikel oder Mikropartikel, die daran angefügte Targetmoleküle aufweisen, gebunden ist;
b) das Durchführen einer ersten Runde magnetischer Trennung durch Verwenden eines magnetischen Stands zum Auffangen des ersten Teils der mehreren Oligonucleotide, die an die mehreren magnetischen Nanopartikel oder Mikropartikel, die daran angefügte Targetmoleküle aufweisen, gebunden sind;
c) das Dispergieren des ersten Teils der mehreren Oligonucleotide, die an die mehreren magnetischen Nanopartikel oder Mikropartikel, die daran angefügte Targetmoleküle aufweisen, gebunden sind, in einem bindenden Puffer;
d) das Durchführen eines magnetisch unterstützten Screenens durch Aufbringen eines oszillierenden Magnetfelds auf den ersten Teil der mehreren Oligonucleotide, die an die mehreren magnetischen Nanopartikel oder Mikropartikel, die daran angefügte Targetmoleküle aufweisen, gebunden sind, so dass ein Teil des ersten Teils der mehreren Oligonucleotide, von den mehreren magnetischen Nanopartikeln oder Mikropartikeln, die daran angefügte Targetmoleküle aufweisen, abgetrennt wird;
e) das Durchführen einer zweiten Runde magnetischer Trennung durch Verwenden des magnetischen Stands zum Auffangen eines zweiten Teils des ersten Teils der mehreren Oligonucleotide, die an die mehreren magnetischen Nanopartikel oder Mikropartikel, die daran angefügte Targetmoleküle aufweisen, nach Schritt d) gebunden geblieben sind, so dass der Teil des ersten Teils der mehreren Oligonucleotide, der von den mehreren magnetischen Nanopartikel oder Mikropartikel, die daran angefügte Targetmoleküle aufweisen, abgesondert ist, entfernt wird;
f) das Dispergieren des zweiten Teils des ersten Teils der mehreren Oligonucleotide, die an die mehreren magnetischen Nanopartikel oder Mikropartikel, die daran angefügte Targetmoleküle aufweisen, nach Schritt d) gebunden geblieben sind, in einem bindenden Puffer;
g) das Eluieren des zweiten Teils des ersten Teils der mehreren Oligonucleotide, die an die mehreren magnetischen Nanopartikel oder Mikropartikel, die daran angefügte Targetmoleküle aufweisen, nach Schritt d) gebunden geblieben sind, von den mehreren magnetischen Nanopartikeln oder Mikropartikeln, an die Targetmoleküle angefügt sind;
h) das Durchführen einer dritten Runde magnetischer Trennung durch Verwenden des magnetischen Stands zum Entfernen der mehreren magnetischen Nanopartikel oder Mikropartikel, die daran angefügter Targetmoleküle aufweisen, von dem eluierten zweiten Teil des ersten Teils der mehreren Oligonucleotide; und
i) das Durchführen einer negativen Auswahl des eluierten zweiten Teils des ersten Teils der mehreren Oligonucleotide, um Aptamere auszuwählen, die für die Targetmoleküle spezifisch sind, wobei die Aptamere, die für die Targetmoleküle spezifisch sind, nicht an die mehreren magnetischen Nanopartikel oder Mikropartikel gebunden sind, ohne dass die Targetmoleküle daran angefügt sind,
wobei die Targetmoleküle an die mehreren magnetischen Nanopartikel oder Mikropartikel durch Konjugationspaare angefügt sind, wobei die Konjugationspaare erste Komponenten umfassen, die an die mehreren magnetischen Nanopartikel oder Mikropartikel angeheftet sind und zweite Komponenten an die Targetmoleküle angeheftet sind und
wobei der Schritt i) des Durchführens der negativen Auswahl Folgendes umfasst:
das Zugeben von mehreren magnetischen Nanopartikeln oder Mikropartikeln, bei denen die ersten Komponenten daran angeheftet sind, um den zweiten Teil des ersten Teils der mehreren Oligonucleotide zu eluieren:
das Inkubieren der mehreren magnetischen Nanopartikel oder Mikropartikel, bei denen die die ersten Komponenten daran mit dem eluierten zweiten Teil des ersten Teils der mehreren Oligonucleotide angeheftet sind; und
das Durchführen einer vierten Runde magnetischer Trennung, um die Aptamere, die für die Targetmoleküle spezifisch sind, durch Entfernen der mehreren Oligonucleotide, die an die mehreren magnetischen Nanopartikel oder Mikropartikel gebunden sind, bei denen die ersten Komponenten angeheftet sind, zu erhalten.

2. Aptamerselektionsprotokoll nach Anspruch 1, wobei das oszillierende Magnetfeld ein rotierendes Magnetfeld ist.

3. Aptamerselektionsprotokoll nach Anspruch 2, wobei die Frequenz des rotierenden Magnetfelds 1 Hz bis 300 KHz beträgt.

4. Aptamerselektionsprotokoll nach Anspruch 2, wobei die Stärke des rotierenden Magnetfelds 5 Gauss bis 1000 Gauss beträgt.

5. Aptamerselektionsprotokoll nach Anspruch 1, wobei das oszillierende Magnetfeld ein wechselndes Magnetfeld ist.

6. Aptamerselektionsprotokoll nach Anspruch 5, wobei die Frequenz des wechselnden Magnetfelds 1 Hz bis 300 KHz beträgt.

7. Aptamerselektionsprotokoll nach Anspruch 5, wobei die Stärke des wechselnden Magnetfelds 5 Gauss bis 1000 Gauss beträgt.

8. Aptamerselektionsprotokollna1 1, wobei die Größe des magnetischen Nanopartikels 5 nm bis 500 nm beträgt.

9. Aptamerselektionsprotokoll nach Anspruch 1, wobei die Größe des magnetischen Mikropartikels 0,50 µm bis 50 µm beträgt.

10. Aptamerselektionsprotokoll umfassend:
a) das Bereitstellen einer Probe umfassend mindestens eine Zufalls-Oligonucleotid-Bibliothek, die mehrere Oligonucleotide umfasst, und das Verdünnen mit einem bindenden Puffer;
b) das Durchführen einer negativen Selektion an der Probe durch Inkubieren mit mehreren ersten magnetischen Nanopartikeln oder Mikropartikeln, ohne dass Targetmoleküle daran angefügt sind, zum Isolieren eines ersten Teils der mehreren Oligonucleotide, die nicht an die mehreren ersten magnetischen Nanopartikel oder Mikropartikel gebunden sind, ohne dass Targetmoleküle daran angefügt ;
c) das Durchführen einer ersten Runde magnetischer Trennung, um die mehreren Oligonucleotide, die an die mehreren ersten magnetischen Nanopartikel oder Mikropartikel gebunden sind, ohne dass die Targetmoleküle daran angefügt sind, von dem ersten Teil der mehreren Oligonucleotide zu entfernen;
d) das Inkubieren des ersten Teils der mehreren Oligonucleotide mit mehreren zweiten magnetischen Nanopartikeln oder Mikropartikeln, an die die Targetmoleküle angefügt sind, wobei ein zweiter Teil des ersten Teils der mehreren Oligonucleotide an die mehreren magnetischen Nanopartikel oder Mikropartikel, die daran angefügte Targetmoleküle aufweisen, gebunden ist:
e) das Durchführen einer zweiten Runde magnetischer Trennung zum Auffangen des zweiten Teils des ersten Teils der mehreren Oligonucleotide, die an die mehreren zweiten magnetischen Nanopartikel oder Mikropartikel, die daran angefügte Targetmoleküle aufweisen, gebunden sind;
f) das Dispergieren des zweiten Teils des ersten Teils der mehreren Oligonucleotide, die an die mehreren magnetischen Nanopartikel oder Mikropartikel, die daran angefügte Targetmoleküle aufweisen, gebunden sind, in einem bindenden Puffer;
g) das Durchführen eines magnetisch unterstützten Screenens durch Aufbringen eines oszillierenden Magnetfelds auf den zweiten Teil des ersten Teils der mehreren Oligonucleotide, die an die mehreren zweiten magnetischen Nanopartikel oder Mikropartikel, die daran angefügter Targetmoleküle aufweisen, gebunden sind, so dass ein Teil des zweiten Teils des ersten Teils der mehreren Oligonucleotide von den mehreren zweiten magnetischen Nanopartikeln oder Mikropartikeln, die daran angefügte Targetmoleküle aufweisen, abgelöst wird;
h) das Durchführen einer dritten Runde magnetischer Trennung durch Verwenden eines magnetischen Stands zum Auffangen eines dritten Teils des zweiten Teils des ersten Teils der mehreren Oligonucleotide, die an die mehreren zweiten magnetischen Nanopartikel oder Mikropartikel, die daran angefügter Targetmoleküle aufweisen, nach Schritt g) gebunden geblieben sind, so dass der Teil des zweiten Teils des ersten Teils der mehreren Oligonucleotide, die von den mehreren magnetischen Nanopartikel oder Mikropartikel, die daran angefügte Targetmoleküle aufweisen, abgelöst ist, entfernt wird;
i) das Dispergieren des dritten Teils des zweiten Teils des ersten Teils der mehreren Oligonucleotide, die an die mehreren zweiten magnetischen Nanopartikel oder Mikropartikel, die daran angefügte Targetmoleküle aufweisen, nach Schritt g) gebunden geblieben sind, mit ddH₂O;
j) das Eluieren des dritten Teils des zweiten Teils des ersten Teils der mehreren Oligonucleotide, die an die mehreren zweiten magnetischen Nanopartikel oder Mikropartikel, die daran angefügte Targetmoleküle aufweisen, nach Schritt g) gebunden geblieben sind, von den mehreren zweiten magnetische Nanopartikeln oder Mikropartikeln, die daran angefügte Targetmoleküle aufweisen; und
k) das Durchführen einer vierten Runde magnetischer Trennung, um die mehreren zweiten magnetischen Nanopartikel oder Mikropartikel, die daran angefügte Targetmoleküle aufweisen, von dem eluierten dritten Teil des zweiten Teils des ersten Teils der mehreren Oligonucleotide zu entfernen und Aptamere, die für die Targetmoleküle spezifisch sind, zu erhalten.
wobei die Targetmoleküle an die mehreren zweiten magnetischen Nanopartikel oder Mikropartikel durch Konjugationspaare angefügt sind und die Konjugationspaare erste Komponenten umfassen, die an die mehreren zweiten magnetischen Nanopartikel oder Mikropartikel angeheftet sind und zweite Komponenten an die Targetmoleküle angeheftet sind und bei den mehreren der ersten magnetischen Nanopartikel oder Mikropartikel die ersten Komponenten daran angeheftet aufweisen.

11. Aptamerselektionsprotokoll nach Anspruch 10, wobei das oszillierende Magnetfeld ein rotierendes Magnetfeld ist.

12. Aptamerselektionsprotokoll nach Anspruch 11, wobei die Frequenz des rotierenden Magnetfelds 1 Hz bis 300 KHz beträgt.

13. Aptamerselektionsprotokoll nach Anspruch 11, wobei die Stärke des rotierenden Magnetfelds 5 Gauss bis 1000 Gauss beträgt.

14. Aptamerselektionsprotokoll nach Anspruch 10, wobei das oszillierende Magnetfeld ein wechselndes Magnetfeld ist.

15. Aptamerselektionsprotokoll nach Anspruch 14, wobei die Frequenz des wechselnden Magnetfelds 1 Hz bis 300 KHz beträgt.

16. Aptamerselektionsprotokoll nach Anspruch 14, wobei die Stärke des wechselnden Magnetfelds 5 Gauss bis 1000 Gauss beträgt.

17. Aptamerselektionsprotokoll nach Anspruch 10, wobei die Größe des ersten oder zweien magnetischen Nanopartikels 5 nm bis 500 nm beträgt.

18. Aptamerselektionsprotokoll nach Anspruch 10, wobei die Größe des ersten oder zweiten magnetischen Mikropartikels 0,50 µm bis 50 µm beträgt.

19. Aptamerselektionsprotokoll mit einem erwünschten Affinitätsbereich des ausgewählten Aptamers zu dem Target, umfassend:
a) das Bereitstellen einer Probe umfassend mindestens eine Zufalls-Oligonucleotid-Bibliothek, die mehrere Oligonucleotide und mehrere magnetische Nanopartikel oder Mikropartikel aufweist, die daran angefügte Targetmoleküle aufweisen, wobei ein erster Teil der mehreren Oligonucleotide an die mehreren magnetischen Nanopartikel oder Mikropartikel, die daran angefügte Targetmoleküle aufweisen, gebunden ist;
b) das Durchführen einer ersten Runde magnetischer Trennung durch Verwenden eines magnetischen Stands zum Auffangen des ersten Teils der mehreren Oligonucleotide, die an die mehreren magnetischen Nanopartikel oder Mikropartikel, die daran angefügte Targetmoleküle aufweisen, gebunden sind;
c) das Dispergieren des ersten Teils der mehreren Oligonucleotide, die an die mehreren magnetischen Nanopartikel oder Mikropartikel, die daran angefügte Targetmoleküle aufweisen, gebunden sind, in einem bindenden Puffer;
d) das Durchführen eines magnetisch unterstützten Screenens durch Aufbringen eines oszillierenden Magnetfelds auf den ersten Teil der mehreren Oligonucleotide, die an die mehreren magnetischen Nanopartikel oder Mikropartikel, die daran angefügte Targetmoleküle aufweisen, gebunden sind, so dass ein Teil des ersten Teils der mehreren Oligonucleotide, von den mehreren magnetischen Nanopartikeln oder Mikropartikeln, die daran angefügte Targetmoleküle aufweisen, abgelöst wird;
e) das Durchführen einer zweiten Runde magnetischer Trennung durch Verwenden des magnetischen Stands zum Auffangen eines zweiten Teils des ersten Teils der mehreren Oligonucleotide, die an die mehreren magnetischen Nanopartikel oder Mikropartikel, die daran angefügte Targetmoleküle aufweisen, nach Schritt d) gebunden geblieben sind, so dass der Teil des ersten Teils der mehreren Oligonucleotide, der von den mehreren magnetischen Nanopartikel oder Mikropartikel, die daran angefügte Targetmoleküle aufweisen, abgelöst ist, entfernt wird;
f) das Dispergieren des zweiten Teils des ersten Teils der mehreren Oligonucleotide, die an die mehreren magnetischen Nanopartikel oder Mikropartikel, die daran angefügte Targetmoleküle aufweisen, nach Schritt d) gebunden geblieben sind, in einem bindenden Puffer;
g) das Durchführen eines zweiten magnetisch unterstützten Screenens durch Aufbringen eines zweiten oszillierenden Magnetfelds, das eine Feldfrequenz und/oder Feldstärke aufweist, die höher sind als diejenigen des oszillierenden Magnetfelds, auf den zweiten Teil des ersten Teils der mehreren Oligonucleotide, die an die mehreren magnetischen Nanopartikel oder Mikropartikel, die daran angefügte Targetmoleküle aufweisen, nach Schritt d) gebunden geblieben sind, so dass ein Teil des zweiten Teils des ersten Teils der mehreren Oligonucleotide, die in Schritt d) gebunden geblieben sind, von den mehreren magnetischen Nanopartikeln oder Mikropartikeln, die daran angefügte Targetmoleküle aufweisen, abgelöst wird;
h) das Durchführen einer dritten Runde magnetischer Trennung durch Verwenden des magnetischen Stands zum Auffangen eines dritten Teils des zweiten Teils des ersten Teils der mehreren Oligonucleotide, die von den mehreren magnetischen Nanopartikeln oder Mikropartikeln, die daran angefügte Targetmoleküle aufweisen, abgelöst sind;
i) das Durchführen einer negativen Auswahl des dritten Teils des zweiten Teils des ersten Teils der mehreren Oligonucleotide, um Aptamere auszuwählen, die für die Targetmoleküle spezifisch sind, wobei die Aptamere, die für die Targetmoleküle spezifisch sind, mit der erwünschten Affinität nicht an die mehreren magnetischen Nanopartikel oder Mikropartikel gebunden sind, ohne dass die Targetmoleküle daran angefügt sind,
wobei die Targetmoleküle an die mehreren magnetischen Nanopartikel oder Mikropartikel durch Konjugationspaare angefügt sind und die Konjugationspaare erste Komponenten umfassen, die an die mehreren magnetischen Nanopartikel oder Mikropartikel angeheftet sind und zweite Komponenten an die Targetmoleküle angeheftet sind und
wobei der Schritt i) des Durchführens der negativen Auswahl Folgendes umfasst:
das Zugeben von mehreren magnetischen Nanopartikeln oder Mikropartikeln, bei denen die ersten Komponenten daran angeheftet sind;
das Inkubieren der mehreren magnetischen Nanopartikel oder Mikropartikel, bei denen die die ersten Komponenten daran angeheftet sind, mit dritten Teil des zweiten Teils des ersten Teils der mehreren Oligonucleotide angeheftet sind; und
das Durchführen einer vierten Runde magnetischer Trennung, um die Aptamere, die für die Targetmoleküle spezifisch sind, durch Entfernen der mehreren Oligonucleotide, die an die mehreren magnetischen Nanopartikel oder Mikropartikel gebunden sind, bei denen die ersten Komponenten angeheftet sind, zu erhalten.
Aptamerselektionsprotokoll nach Anspruch 1, wobei das oszillierende Magnetfeld ein rotierendes Magnetfeld ist.

20. Aptamerselektionsprotokoll nach Anspruch 19, wobei die ersten und zweiten oszillierenden Magnetfelder rotierende Magnetfelder sind.

21. Aptamerselektionsprotokoll nach Anspruch 20, wobei die Feldfrequenz des rotierenden Magnetfelds 1 Hz bis 300 KHz beträgt.

22. Aptamerselektionsprotokoll nach Anspruch 20, wobei die Feldstärke des rotierenden Magnetfelds 5 Gauss bis 1000 Gauss beträgt.

23. Aptamerselektionsprotokoll nach Anspruch 19, wobei die ersten und zweiten oszillierenden Magnetfelder wechselnde Magnetfelder sind.

24. Aptamerselektionsprotokoll nach Anspruch 23, wobei die Feldfrequenz der wechselnden Magnetfelder 1 Hz bis 300 KHz beträgt.

25. Aptamerselektionsprotokoll nach Anspruch 23, wobei die Feldstärke der wechselnden Magnetfelder 5 Gauss bis 1000 Gauss beträgt.

26. Aptamerselektionsprotokoll nach Anspruch 1, wobei die Größe des magnetischen Nanopartikels 5 nm bis 500 nm beträgt.

27. Aptamerselektionsprotokoll nach Anspruch 19, wobei die Größe des magnetischen Mikropartikels 0,50 µm bis 50 µm beträgt.

## Revendications

1. Protocole de sélection d'aptamères, consistant à :
a) fournir un échantillon comprenant au moins une bibliothèque d'oligonucléotides aléatoire possédant une pluralité d'oligonucléotides et une pluralité de nanoparticules ou de microparticules magnétiques ayant des molécules cibles jointes sur celles-ci, une première partie de la pluralité d'oligonucléotides étant liée à la pluralité de nanoparticules ou de microparticules magnétiques ayant les molécules cibles jointes sur celles-ci ;
b) effectuer un premier tour de séparation magnétique en utilisant un support magnétique pour recueillir la première partie de la pluralité d'oligonucléotides liés à la pluralité de nanoparticules ou de microparticules magnétiques ayant les molécules cibles jointes sur celles-ci ;
c) disperser la première partie de la pluralité d'oligonucléotides liés à la pluralité de nanoparticules ou de microparticules magnétiques ayant les molécules cibles jointes sur celles-ci dans un tampon de liaison ;
d) effectuer un criblage à assistance magnétique en appliquant un champ magnétique à oscillation à la première partie de la pluralité d'oligonucléotides liés à la pluralité de nanoparticules ou de microparticules magnétiques ayant les molécules cibles jointes sur celles-ci, de sorte qu'une partie de la première partie de la pluralité d'oligonucléotides soit détachée de la pluralité de nanoparticules ou de microparticules magnétiques ayant les molécules cibles jointes sur celles-ci ;
e) effectuer un second tour de séparation magnétique en utilisant le support magnétique pour recueillir une seconde partie de la première partie de la pluralité d'oligonucléotides qui restait liée à la pluralité de nanoparticules ou de microparticules magnétiques ayant les molécules cibles jointes sur celle-ci après l'étape d), de sorte que la partie de la première partie de la pluralité d'oligonucléotides détachés de la pluralité de nanoparticules ou de microparticules magnétiques ayant les molécules cibles jointes sur celles-ci soit éliminée ;
f) disperser la seconde partie de la première partie de la pluralité d'oligonucléotides qui restait liée à la pluralité de nanoparticules ou de microparticules magnétiques ayant les molécules cibles jointes sur celles-ci après l'étape d) dans un tampon de liaison ;
g) éluer la seconde partie de la première partie de la pluralité d'oligonucléotides qui restait liée à la pluralité de nanoparticules ou de microparticules magnétiques ayant les molécules cible jointes sur celles-ci après l'étape d) à partir de la pluralité de nanoparticules ou de microparticules magnétiques ayant les molécules cibles jointes sur celles-ci ;
h) effectuer un troisième tour de séparation magnétique en utilisant le support magnétique pour éliminer la pluralité de nanoparticules ou de microparticules magnétiques ayant les molécules cibles jointes sur celles-ci à partir de la seconde partie éluée et de la première partie de la pluralité d'oligonucléotides ; et
i) effectuer une sélection négative sur la seconde partie éluée de la première partie de la pluralité d'oligonucléotides pour sélectionner les aptamères spécifiques aux molécules cibles, les aptamères spécifiques aux molécules cibles ne sont pas liés à la pluralité de nanoparticules ou de microparticules magnétiques sans avoir les molécules cibles liées sur celles-ci,
dans lequel les molécules cibles sont jointes à la pluralité de nanoparticules ou de microparticules magnétiques à travers des paires de conjugaison, les paires de conjugaison incluent des premiers composants attachés à la pluralité de nanoparticules ou de microparticules magnétiques et des seconds composants attachés aux molécules cibles, et
dans lequel l'étape i) effectuant la sélection négative consiste à :
ajouter une pluralité de nanoparticules ou de microparticules magnétiques ayant les premiers composants attachés sur celles-ci à la seconde partie éluée de la première partie de la pluralité d'oligonucléotides ;
incuber la pluralité de nanoparticules ou de microparticules magnétiques ayant les premiers composants attachés sur celles-ci avec la seconde partie éluée de la première partie de la pluralité d'oligonucléotides ; et
effectuer un quatrième tour de séparation magnétique pour obtenir les aptamères spécifiques aux molécules cibles en éliminant la pluralité d'oligonucléotides liés à la pluralité de nanoparticules ou de microparticules magnétiques ayant les premiers composants attachés sur celles-ci.

2. Protocole de sélection d'aptamères selon la revendication 1, dans lequel le champ magnétique à oscillation est un champ magnétique tournant.

3. Protocole de sélection d'aptamères selon la revendication 2, dans lequel la fréquence du champ magnétique tournant est comprise entre 1 Hz et 300 kHz.

4. Protocole de sélection d'aptamères selon la revendication 2, dans lequel la force du champ magnétique tournant est comprise entre 5 gauss et 1000 gauss.

5. Protocole de sélection d'aptamères selon la revendication 1, dans lequel le champ magnétique à oscillation est un champ magnétique alternant.

6. Protocole de sélection d'aptamères selon la revendication 5, dans lequel la fréquence du champ magnétique alternant est comprise entre 1 Hz et 300 kHz.

7. Protocole de sélection d'aptamères selon la revendication 5, dans lequel la force du champ magnétique alternant est comprise entre 5 gauss et 1000 gauss.

8. Protocole de sélection d'aptamères selon la revendication 1, dans lequel la taille de la nanoparticule magnétique est comprise entre 5 nm et 500 nm.

9. Protocole de sélection d'aptamères selon la revendication 1, dans lequel la taille de la microparticule magnétique est comprise entre 0,50 µm et 50 µm.

10. Protocole de sélection d'aptamères, consistant à :
a) fournir un échantillon comprenant au moins une bibliothèque d'oligonucléotides aléatoire ayant une pluralité d'oligonucléotides et diluer avec un tampon de liaison ;
b) effectuer une sélection négative sur l'échantillon en incubant avec une pluralité de premières nanoparticules ou microparticules magnétiques sans avoir les molécules cibles jointes sur celles-ci pour isoler une première partie de la pluralité d'oligonucléotides non liés à la pluralité de premières nanoparticules ou de microparticules magnétiques sans avoir les molécules cibles jointes sur celles-ci ;
c) effectuer un premier tour de séparation magnétique pour éliminer de la pluralité d'oligonucléotides liés à la pluralité de premières nanoparticules ou microparticules magnétiques sans avoir les molécules cibles jointes sur celles-ci à partir de la première partie de la pluralité d'oligonucléotides ;
d) incuber la première partie de la pluralité d'oligonucléotides avec une pluralité de secondes nanoparticules ou microparticules magnétiques ayant les molécules cibles jointes sur celles-ci, la seconde partie de la première partie de la pluralité d'oligonucléotides étant liée à la pluralité de nanoparticules ou de microparticules magnétiques ayant les molécules cibles jointes sur celles-ci ;
e) effectuer un second tour de séparation magnétique pour recueillir la seconde partie de la première partie de la pluralité d'oligonucléotides liée à la pluralité de secondes nanoparticules ou microparticules magnétiques ayant les molécules cibles jointes sur celles-ci ;
f) disperser la seconde partie de la première partie de la pluralité d'oligonucléotides liée à la pluralité de secondes nanoparticules ou microparticules magnétiques ayant les molécules cibles jointes sur celles-ci dans un tampon de liaison ;
g) effectuer un criblage à assistance magnétique en appliquant un champ magnétique à oscillation sur la seconde partie de la première partie de la pluralité d'oligonucléotides liés à la pluralité de secondes nanoparticules ou microparticules magnétiques ayant les molécules cibles jointes sur celles-ci, de sorte qu'une partie de la seconde partie des secondes nanoparticules ou microparticules magnétiques soit détachée de la pluralité des secondes nanoparticules ou microparticules magnétiques ayant les molécules cibles jointes sur celles-ci ;
h) effectuer un troisième tour de séparation magnétique en utilisant un support magnétique pour recueillir une troisième partie de la seconde partie de la première partie de la pluralité d'oligonucléotides qui restait liée à la pluralité de secondes nanoparticules ou microparticules magnétiques ayant les molécules cibles jointes sur celles-ci après l'étape g), de sorte que la partie de la seconde partie de la première partie de la pluralité d'oligonucléotides détachée de la pluralité de secondes nanoparticules ou microparticules magnétiques ayant les molécules cibles jointes sur celles-ci soit éliminée ;
i) disperser la troisième partie de la seconde partie de la première partie de la pluralité d'oligonucléotides qui restait liée à la pluralité de secondes nanoparticules ou microparticules magnétiques ayant les molécules cibles jointes sur celles-ci après l'étape g) avec ddH₂O ;
j) éluer la troisième partie de la seconde partie de la première partie de la pluralité d'oligonucléotides qui restait liée à la pluralité de secondes nanoparticules ou microparticules magnétiques ayant les molécules cibles jointes sur celles-ci après l'étape g) de la pluralité de secondes nanoparticules ou microparticules magnétiques ayant les molécules cibles jointes sur celles-ci ; et
k) effectuer un quatrième tour de séparation magnétique pour éliminer la pluralité de secondes nanoparticules ou microparticules magnétiques ayant les molécules cibles jointes sur celles-ci à partir de la troisième partie éluée de la seconde partie de la première partie de la pluralité d'oligonucléotides et pour obtenir les aptamères spécifiques aux molécules cibles,
dans lequel les molécules cibles sont jointes à la pluralité de secondes nanoparticules ou microparticules magnétiques à travers des paires de conjugaison, et les paires de conjugaison incluent des premiers composants attachés à la pluralité de secondes nanoparticules ou microparticules magnétiques et des seconds composants attachés aux molécules cibles, et la pluralité de premières nanoparticules ou microparticules magnétiques a les premiers composants attachés sur celles-ci.

11. Protocole de sélection d'aptamères selon la revendication 10, dans lequel le champ magnétique à oscillation est un champ magnétique tournant.

12. Protocole de sélection d'aptamères selon la revendication 11, dans lequel la fréquence du champ magnétique tournant est comprise entre 1 Hz et 300 kHz.

13. Protocole de sélection d'aptamères selon la revendication 11, dans lequel la force du champ magnétique tournant est comprise entre 5 gauss et 1000 gauss.

14. Protocole de sélection d'aptamères selon la revendication 10, dans lequel le champ magnétique à oscillation est un champ magnétique alternant.

15. Protocole de sélection d'aptamères selon la revendication 14, dans lequel la fréquence du champ magnétique alternant est comprise entre 1 Hz et 300 kHz.

16. Protocole de sélection d'aptamères selon la revendication 14, dans lequel la force du champ magnétique alternant est comprise entre 5 gauss et 1000 gauss.

17. Protocole de sélection d'aptamères selon la revendication 10, dans lequel la taille de la première ou de la seconde nanoparticule magnétique est comprise entre 5 nm et 500 nm.

18. Protocole de sélection d'aptamères selon la revendication 10, dans lequel la taille de la première ou de la seconde microparticule magnétique est comprise entre 0,50 µm et 50 µm.

19. Protocole de sélection d'aptamères avec une gamme d'affinité souhaitée de l'aptamère sélectionné à la cible, consistant à :
a) fournir un échantillon comprenant au moins une bibliothèque d'oligonucléotides aléatoire ayant une pluralité d'oligonucléotides et une pluralité de nanoparticules ou de microparticules magnétiques ayant des molécules cibles jointes sur celles-ci, une première partie de la pluralité d'oligonucléotides étant liée à la pluralité de nanoparticules ou de microparticules magnétiques ayant les molécules cibles jointes sur celles-ci ;
b) effectuer un premier tour de séparation magnétique en utilisant un support magnétique pour recueillir la première partie de la pluralité d'oligonucléotides liés à la pluralité de nanoparticules ou de microparticules magnétiques ayant les molécules cibles jointes sur celles-ci ;
c) disperser la première partie de la pluralité d'oligonucléotides liés à la pluralité de nanoparticules ou de microparticules magnétiques ayant les molécules cibles jointes sur celles-ci dans un tampon de liaison ;
d) effectuer un premier criblage à assistance magnétique en appliquant un premier champ magnétique à oscillation à la première partie de la pluralité d'oligonucléotides liés à la pluralité de nanoparticules ou de microparticules magnétiques ayant les molécules cibles jointes sur celles-ci, de sorte qu'une partie de la première partie de la pluralité d'oligonucléotides soit détachée de la pluralité de nanoparticules ou de microparticules magnétiques ayant les molécules cibles jointes sur celles-ci ;
e) effectuer un second tour de séparation magnétique en utilisant le support magnétique pour recueillir une seconde partie de la première partie de la pluralité d'oligonucléotides qui restait liée à la pluralité de nanoparticules ou de microparticules magnétiques ayant les molécules cibles jointes sur celle-ci après l'étape d), de sorte que la partie de la première partie de la pluralité d'oligonucléotides détachés de la pluralité de nanoparticules ou de microparticules magnétiques ayant les molécules cibles jointes sur celles-ci soit éliminée ;
f) disperser la seconde partie de la première partie de la pluralité d'oligonucléotides qui restait liée à la pluralité de nanoparticules ou de microparticules magnétiques ayant les molécules cibles jointes sur celles-ci après l'étape d) dans un tampon de liaison ;
g) effectuer un second criblage à assistance magnétique en appliquant un second champ magnétique à oscillation ayant une fréquence de champ et/ou une force de champ supérieures à celles du premier champ magnétique à oscillation sur la seconde partie de la première partie de la pluralité d'oligonucléotides qui restait liée à la pluralité de nanoparticules ou de microparticules magnétiques ayant les molécules cible jointes sur celles-ci après l'étape d), de sorte qu'une partie de la seconde partie de la première partie de la pluralité d'oligonucléotides qui restait liée dans l'étape d) soit détachée de la pluralité de nanoparticules ou de microparticules magnétiques ayant les molécules cibles jointes sur celles-ci ;
h) effectuer un troisième tour de séparation magnétique en utilisant le support magnétique pour recueillir une troisième partie de la seconde partie de la première partie de la pluralité d'oligonucléotides détachés de la pluralité de nanoparticules ou de microparticules magnétiques ayant les molécules cibles jointes sur celles-ci ; et
i) effectuer une sélection négative sur la troisième partie de la seconde partie de la première partie de la pluralité d'oligonucléotides pour sélectionner les aptamères spécifiques aux molécules cibles, les aptamères spécifiques aux molécules cibles avec l'affinité souhaitable ne sont pas liés à la pluralité de nanoparticules ou de microparticules magnétiques sans avoir les molécules cibles liées sur celles-ci,
dans lequel les molécules cibles sont jointes à la pluralité de nanoparticules ou de microparticules magnétiques à travers des paires de conjugaison, et les paires de conjugaison incluent des premiers composants attachés à la pluralité de nanoparticules ou de microparticules magnétiques et des seconds composants attachés aux molécules cibles, et
dans lequel l'étape i) effectuant la sélection négative consiste à :
ajouter une pluralité de nanoparticules ou de microparticules magnétiques ayant les premiers composants attachés sur celles-ci ;
incuber la pluralité de nanoparticules ou de microparticules magnétiques ayant les premiers composants attachés sur celles-ci avec la troisième partie de la seconde partie de la première partie de la pluralité d'oligonucléotides ; et
effectuer un quatrième tour de séparation magnétique pour obtenir les aptamères spécifiques aux molécules cibles en éliminant la pluralité d'oligonucléotides liés à la pluralité de nanoparticules ou de microparticules magnétiques ayant les premiers composants attachés sur celles-ci.

20. Protocole de sélection d'aptamères selon la revendication 19, dans lequel les premier et second champs magnétiques à oscillation sont des champs magnétiques tournants.

21. Protocole de sélection d'aptamères selon la revendication 20, dans lequel la fréquence de champ des champs magnétiques tournants est comprise entre 1 Hz et 300 kHz.

22. Protocole de sélection d'aptamères selon la revendication 20, dans lequel la force de champ des champs magnétiques tournants est comprise entre 5 gauss et 1000 gauss.

23. Protocole de sélection d'aptamères selon la revendication 19, dans lequel les premier et second champs magnétiques à oscillation sont des champs magnétiques alternants.

24. Protocole de sélection d'aptamères selon la revendication 23, dans lequel la fréquence de champ des champs magnétiques alternants est comprise entre 1 Hz et 300 kHz.

25. Protocole de sélection d'aptamères selon la revendication 23, dans lequel la force de champ des champs magnétiques alternants est comprise entre 5 gauss et 1000 gauss.

26. Protocole de sélection d'aptamères selon la revendication 19, dans lequel la taille de la nanoparticule magnétique est comprise entre 5 nm et 500 nm.

27. Protocole de sélection d'aptamères selon la revendication 19, dans lequel la taille de la microparticule magnétique est comprise entre 0,50 µm et 50 µm.


### SEQUENCE LISTING

<110> Hong, Chin-Yih
<120> MAGNETIC-ASSISTED RAPID APTAMER SELECTION METHOD FOR GENERATING HIGH AFFINITY DNA APTAMER
<130> 47132-US-PA
<160> 59
<170> PatentIn version 3.5
<210> 1
   <211> 20
   <212> DNA
   <213> Artificial Sequence
<220>
   <223> Primer
<400> 1
   agcagcacag aggtcagatg 20
<210> 2
   <211> 20
   <212> DNA
   <213> Artificial Sequence
<220>
   <223> Primer
<400> 2
   ttcacggtag cacgcatagg 20
<210> 3
   <211> 20
   <212> DNA
   <213> Artificial Sequence
<220>
   <223> Primer
<400> 3
   taatacgact cactataggg 20
<210> 4
   <211> 20
   <212> DNA
   <213> Artificial Sequence
<220>
   <223> Randomized synthesized DNA sequence
<400> 4
   agacctttga aggccccaag 20
<210> 5
   <211> 20
   <212> DNA
   <213> Artificial Sequence
<220>
   <223> Randomized synthesized DNA sequence
<400> 5
   ttggtttgta acattgatgg 20
<210> 6
   <211> 20
   <212> DNA
   <213> Artificial Sequence
<220>
   <223> Randomized synthesized DNA sequence
<400> 6
   agttagtttc agtgggtccc 20
<210> 7
   <211> 20
   <212> DNA
   <213> Artificial Sequence
<220>
   <223> Randomized synthesized DNA sequence
<400> 7
   accgtgttgt ctgaggtctc 20
<210> 8
   <211> 20
   <212> DNA
   <213> Artificial Sequence
<220>
   <223> Randomized synthesized DNA sequence
<400> 8
   tcggtgtgcc actttctcaa 20
<210> 9
   <211> 20
   <212> DNA
   <213> Artificial Sequence
<220>
   <223> Randomized synthesized DNA sequence
<400> 9
   ttttagttgt gcctggtgcg 20
<210> 10
   <211> 20
   <212> DNA
   <213> Artificial Sequence
<220>
   <223> Randomized synthesized DNA sequence
<400> 10
   ggtgtgcgtt attctgtggc 20
<210> 11
   <211> 20
   <212> DNA
   <213> Artificial Sequence
<220>
   <223> Randomized synthesized DNA sequence
<400> 11
   ctcttggaca actggattag 20
<210> 12
   <211> 20
   <212> DNA
   <213> Artificial Sequence
<220>
   <223> Randomized synthesized DNA sequence
<400> 12
   accctacgta cgttctcatg 20
<210> 13
   <211> 20
   <212> DNA
   <213> Artificial Sequence
<220>
   <223> Randomized synthesized DNA sequence
<400> 13
   atataatttt gctctttatt 20
<210> 14
   <211> 20
   <212> DNA
   <213> Artificial Sequence
<220>
   <223> Randomized synthesized DNA sequence
<400> 14
   gcccccgaag ttgcttagtc 20
<210> 15
   <211> 20
   <212> DNA
   <213> Artificial Sequence
<220>
   <223> Randomized synthesized DNA sequence
<400> 15
   cacattaggt gtgataaggc 20
<210> 16
   <211> 20
   <212> DNA
   <213> Artificial Sequence
<220>
   <223> Randomized synthesized DNA sequence
<400> 16
   tctgtaattt atagttccat 20
<210> 17
   <211> 20
   <212> DNA
   <213> Artificial Sequence
<220>
   <223> Randomized synthesized DNA sequence
<400> 17
   tttttcgctg gtgttcgacc 20
<210> 18
   <211> 20
   <212> DNA
   <213> Artificial Sequence
<220>
   <223> Randomized synthesized DNA sequence
<400> 18
   attgttcgtt attgcgttcg 20
<210> 19
   <211> 20
   <212> DNA
   <213> Artificial Sequence
<220>
   <223> Randomized synthesized DNA sequence
<400> 19
   atactcgccg cttttgaatt 20
<210> 20
   <211> 20
   <212> DNA
   <213> Artificial Sequence
<220>
   <223> Randomized synthesized DNA sequence
<400> 20
   tatgcgggtg tttgcgactg 20
<210> 21
   <211> 19
   <212> DNA
   <213> Artificial Sequence
<220>
   <223> Randomized synthesized DNA sequence
<400> 21
   ctttctttct aggtgtgtg 19
<210> 22
   <211> 20
   <212> DNA
   <213> Artificial Sequence
<220>
   <223> Randomized synthesized DNA sequence
<400> 22
   tgacttacat gtactacttt 20
<210> 23
   <211> 20
   <212> DNA
   <213> Artificial Sequence
<220>
   <223> Randomized synthesized DNA sequence
<400> 23
   tgtcagtttg atctgtaatt 20
<210> 24
   <211> 20
   <212> DNA
   <213> Artificial Sequence
<220>
   <223> Randomized synthesized DNA sequence
<400> 24
   ctccttttga tttgcgggta 20
<210> 25
   <211> 20
   <212> DNA
   <213> Artificial Sequence
<220>
   <223> Randomized synthesized DNA sequence
<400> 25
   ttggctttaa ctatatggag 20
<210> 26
   <211> 20
   <212> DNA
   <213> Artificial Sequence
<220>
   <223> Randomized synthesized DNA sequence
<400> 26
   tctacgcgat attctccccc 20
<210> 27
   <211> 20
   <212> DNA
   <213> Artificial Sequence
<220>
   <223> Randomized synthesized DNA sequence
<400> 27
   tcttccgccg taccgttccc 20
<210> 28
   <211> 20
   <212> DNA
   <213> Artificial Sequence
<220>
   <223> Randomized synthesized DNA sequence
<400> 28
   gcattgtatc acaggtactg 20
<210> 29
   <211> 20
   <212> DNA
   <213> Artificial Sequence
<220>
   <223> Randomized synthesized DNA sequence
<400> 29
   ttgtctctgg ggatctaaac 20
<210> 30
   <211> 22
   <212> DNA
   <213> Artificial Sequence
<220>
   <223> Randomized synthesized DNA sequence
<400> 30
   tcctgttgat gtgtgttata tg 22
<210> 31
   <211> 20
   <212> DNA
   <213> Artificial Sequence
<220>
   <223> Randomized synthesized DNA sequence
<400> 31
   aattacaaat ttggactgtt 20
<210> 32
   <211> 20
   <212> DNA
   <213> Artificial Sequence
<220>
   <223> Randomized synthesized DNA sequence
<400> 32
   ttgcatagtt cggttctatg 20
<210> 33
   <211> 20
   <212> DNA
   <213> Artificial Sequence
<220>
   <223> Randomized synthesized DNA sequence
<400> 33
   tgtatggtgg ttccatcttc 20
<210> 34
   <211> 20
   <212> DNA
   <213> Artificial Sequence
<220>
   <223> Randomized synthesized DNA sequence
<400> 34
   tatgtatgga atcacaatcc 20
<210> 35
   <211> 20
   <212> DNA
   <213> Artificial Sequence
<220>
   <223> Randomized synthesized DNA sequence
<400> 35
   ttgcgggatg aagtgtctaa 20
<210> 36
   <211> 20
   <212> DNA
   <213> Artificial Sequence
<220>
   <223> Randomized synthesized DNA sequence
<400> 36
   ttggtattcc tcgcgttttt 20
<210> 37
   <211> 20
   <212> DNA
   <213> Artificial Sequence
<220>
   <223> Randomized synthesized DNA sequence
<400> 37
   gcgtttggca tccctcggtc 20
<210> 38
   <211> 20
   <212> DNA
   <213> Artificial Sequence
<220>
   <223> Randomized synthesized DNA sequence
<400> 38
   ctgtggttgg cgcttggcat 20
<210> 39
   <211> 20
   <212> DNA
   <213> Artificial Sequence
<220>
   <223> Randomized synthesized DNA sequence
<400> 39
   tggacttcat tttaagtatg 20
<210> 40
   <211> 20
   <212> DNA
   <213> Artificial Sequence
<220>
   <223> Randomized synthesized DNA sequence
<400> 40
   gtttactgtt ttattgcgct 20
<210> 41
   <211> 20
   <212> DNA
   <213> Artificial Sequence
<220>
   <223> Randomized synthesized DNA sequence
<400> 41
   tatgataagg tgataacatg 20
<210> 42
   <211> 20
   <212> DNA
   <213> Artificial Sequence
<220>
   <223> Randomized synthesized DNA sequence
<400> 42
   ttggcacaaa gcggtgttac 20
<210> 43
   <211> 20
   <212> DNA
   <213> Artificial Sequence
<220>
   <223> Randomized synthesized DNA sequence
<400> 43
   gtgtcgttcg gtgcatcatg 20
<210> 44
   <211> 20
   <212> DNA
   <213> Artificial Sequence
<220>
   <223> Randomized synthesized DNA sequence
<400> 44
   tgctattcag gtggactatg 20
<210> 45
   <211> 20
   <212> DNA
   <213> Artificial Sequence
<220>
   <223> Randomized synthesized DNA sequence
<400> 45
   tggcaacttt tgaacgcgtt 20
<210> 46
   <211> 60
   <212> DNA
   <213> Artificial Sequence
<220>
   <223> Randomized synthesized DNA sequence
<400> 46
   gcctttgaat tttatttcaa cacgtctttt attgttgtta agttcttgat agtactttaa 60
<210> 47
   <211> 60
   <212> DNA
   <213> Artificial Sequence
<220>
   <223> Randomized synthesized DNA sequence
<400> 47
   ttcgctaagt gttggacttt gtgaagcttc ccagatatac gtttgtgtgc ttggaacttc 60
<210> 48
   <211> 40
   <212> DNA
   <213> Artificial Sequence
<220>
   <223> Randomized synthesized DNA sequence
<400> 48
   ttgtttgttt tgcttgttac tgctttctct tctcttttca 40
<210> 49
   <211> 40
   <212> DNA
   <213> Artificial Sequence
<220>
   <223> Randomized synthesized DNA sequence
<400> 49
   tttatgtgtg ttttgtttat tgctactgtg tctatctatt 40
<210> 50
   <211> 20
   <212> DNA
   <213> Artificial Sequence
<220>
   <223> Randomized synthesized DNA sequence
<400> 50
   tacagtttag tgagtgcaag 20
<210> 51
   <211> 20
   <212> DNA
   <213> Artificial Sequence
<220>
   <223> Randomized synthesized DNA sequence
<400> 51
   ctatgtgagt atttcgccac 20
<210> 52
   <211> 60
   <212> DNA
   <213> Artificial Sequence
<220>
   <223> Randomized synthesized DNA sequence
<400> 52
   tctgttggat aattcttgct taatgtcgaa tactcttaca catcatttct ttcatgcatc 60
<210> 53
   <211> 60
   <212> DNA
   <213> Artificial Sequence
<220>
   <223> Randomized synthesized DNA sequence
<400> 53
   ttatttactg ctcttgtgtc acgttgttta gagctgtact tgctttaata tctggtttaa 60
<210> 54
   <211> 40
   <212> DNA
   <213> Artificial Sequence
<220>
   <223> Randomized synthesized DNA sequence
<400> 54
   taaatttttg tattagtttt tgcattagtt ctgccatact 40
<210> 55
   <211> 40
   <212> DNA
   <213> Artificial Sequence
<220>
   <223> Randomized synthesized DNA sequence
<400> 55
   ttacttgttt cttaactcaa tgagttgttt tgtcatgtcg 40
<210> 56
   <211> 20
   <212> DNA
   <213> Artificial Sequence
<220>
   <223> Randomized synthesized DNA sequence
<400> 56
   gcatgaacca ctaagtgggt 20
<210> 57
   <211> 20
   <212> DNA
   <213> Artificial Sequence
<220>
   <223> Randomized synthesized DNA sequence
<400> 57
   gcctgattca ttgtcggcgc 20
<210> 58
   <211> 20
   <212> DNA
   <213> Artificial Sequence
<220>
   <223> Randomized synthesized DNA sequence
<400> 58
   gcctaaagct ggattccctg 20
<210> 59
   <211> 20
   <212> DNA
   <213> Artificial Sequence
<220>
   <223> Randomized synthesized DNA sequence
<400> 59
   gcctgattca ttgtcggcgc 20